(19) Europäisches Patentamt
European Patent Office
Office européen des brevets

(11) EP 2 272 835 A1

(12) EUROPEAN PATENT APPLICATION
published in accordance with Art. 153(4) EPC

(43) Date of publication:
**12.01.2011 Bulletin 2011/02**

(21) Application number: **09734738.9**

(22) Date of filing: **23.04.2009**

(51) Int Cl.:
***C07D 277/20*** (2006.01) ***A61K 31/426*** (2006.01)
***A61K 31/5377*** (2006.01) ***A61P 1/04*** (2006.01)
***A61P 3/10*** (2006.01) ***A61P 11/00*** (2006.01)
***A61P 11/02*** (2006.01) ***A61P 11/06*** (2006.01)
***A61P 17/00*** (2006.01) ***A61P 17/04*** (2006.01)
***A61P 19/02*** (2006.01) ***A61P 25/00*** (2006.01)
***A61P 27/14*** (2006.01) ***A61P 29/00*** (2006.01)
***A61P 35/04*** (2006.01) ***A61P 37/02*** (2006.01)
***A61P 37/06*** (2006.01) ***A61P 37/08*** (2006.01)
***A61P 43/00*** (2006.01) ***C07D 277/42*** (2006.01)
***C07D 417/10*** (2006.01)

(86) International application number:
**PCT/JP2009/058054**

(87) International publication number:
**WO 2009/131171 (29.10.2009 Gazette 2009/44)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **25.04.2008 JP 2008115993**

(71) Applicant: **Dainippon Sumitomo Pharma Co., Ltd.**
**Osaka 541-8524 (JP)**

(72) Inventors:
- **FUJITA, Hitoshi**
  **Suita-shi**
  **Osaka 564-0053 (JP)**
- **KANAI, Toshio**
  **Suita-shi**
  **Osaka 554-0053 (JP)**
- **IMAI, Satoki**
  **Osaka-shi**
  **Osaka 554-0022 (JP)**

(74) Representative: **Vossius & Partner**
**Siebertstrasse 4**
**81675 München (DE)**

(54) **NOVEL FIVE-MEMBERED RING COMPOUND**

(57) Disclosed is a five-membered ring compound represented by formula (1) or a pharmaceutically acceptable salt thereof. The compound inhibits infiltration of leukocytes including eosinophils and lymphocytes, and is effective as a drug for treating various inflammations. The compound is highly safe and can be administered for a long period. A pharmaceutical product containing the five-membered ring compound or a pharmaceutically acceptable salt thereof is also disclosed.

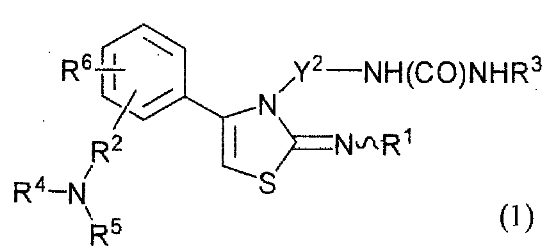

(1)

(In the formula, $R^1$ represents a halogen atom or a phenyl group which may be substituted by a $C_1$-$C_3$ alkyl group or a $C_1$-$C_3$ alkoxy group; $R^2$ represents a $C_1$-$C_3$ alkylene group which may be substituted by a $C_1$-$C_3$ alkyl group or a carbonyl

group; $R^3$ represents a $C_1$-$C_3$ alkyl group; $R^4$ and $R^5$ independently represent a hydrogen atom or a $C_1$-$C_3$ alkyl group, or -N($R^4$)$R^5$ may represent a morpholino group which may be substituted by a $C_1$-$C_3$ alkyl group; $Y^2$ represents a $C_2$-$C_4$ alkylene group; and $R^6$ represents a hydrogen atom, a halogen atom, a $C_1$-$C_3$ alkyl group or a $C_1$-$C_3$ alkoxy group.)

## Description

### TECHNICAL FIELD

The present invention relates to a novel 5-membered ring compound or a pharmaceutically acceptable salt thereof, and a pharmaceutical use thereof. Specifically, the present invention relates to a novel 5-membered ring compound, which is effective for the treatment of various inflammations by binding to a specific binding site of L-threo-3-(3,4-dihydroxyphenyl)-N-[3-(4-fluorophenyl)propyl]serine pyrrolidine amide *in vivo* and inhibiting infiltration of leukocytes including eosinophils, lymphocytes, etc., or a pharmaceutically acceptable salt thereof and a pharmaceutical composition comprising the same.

### BACKGROUND ART

A method for causing immediate asthmatic response (IAR) by administering inhaled allergens to atopic asthma patients has been carried out as an experimental model for dyspnea in bronchial asthma. Specifically, when inhaled allergens have been administered to atopic asthma patients, the patients have had asthmatic response (i.e., bronchial spasm) about 20 minutes after administration and recovered about two hours thereafter. After a continuation of observations of the patients, it has been found that bronchial spasm has been caused again after 6 to 10 hours in about half of the patients who have had the immediate asthmatic response, which has been referred to as Late asthmatic response (LAR). In late asthma, bronchial spasm response has long-continued in association with lung hyperinflation, which has been strongly suppressed by steroid drugs. Therefore, it has been recognized that the bronchial asthma caused by the allergens has been important as a clinical model for dyspnea in steroid-dependent severe bronchial asthma. It has been also recognized that an immediate response has been type I allergy caused as a result of the activation of mast cells by IgE antibody, and a late response has been T lymphocytic and eosinophilic allergy (i.e., eosinophilic inflammations). It has become evident that these immediate and late responses have been also caused by allergic rhinitis and dermatitis. It has been reported that when the late asthmatic response has been caused by allergens in bronchial asthma patients, eosinophils have accumulated in the lungs. Since eosinophilia have been found in blood and expectorated sputum of many bronchial asthma patients, significant numbers of cosinophil infiltration have been found in the lung tissues of patients who have died of asthma, a deposition of major basic protein (MBP) which has been tissue injurious protein derived from eosinophils has been found in bronchial walls and mucus plugs of patients, etc., it has been believed that products derived from eosinophils have played an important role in injuries of airway epithelium associated with late asthma attack.

Steroid drugs have been the only heroic drug against severe bronchial asthma and atopic dermatitis, which such drugs have had strong efficacies as well as adverse effects including hypertension, diabetes, obesity, immune suppression, cataract, mental diseases, atrophia cutis, etc.. Although inhaled steroid drugs have been developed for the purpose of the reduction of the systemic adverse effects, concerns about the adverse effects inherent to inhaled steroid drugs have not been dispelled due to the difficulty in proving that the inhaled steroid drugs have not circulated throughout the body. Recently, since the adverse effects of the inhaled steroid drugs have been reported in Europe and the United States, FDA has instructed to weave letters of warning about the risk of the adverse effects into the packaging insertions of the inhaled steroid drugs for the treatment of bronchial asthma and nasal steroid drugs for the treatment of allergic rhinitis.

As mentioned above, infiltration of eosinophils into involved sites has played an important role in development and degradation of the late response of bronchial asthma as well as allergic dermatitis and rhinitis. However, a heroic drug for the treatment of allergy diseases including bronchial asthma by inhibiting infiltration and activation of eosinophils has been steroid drugs only, and orally-available anti-inflammatory drugs with fewer adverse effects which may be substituted for the steroid drugs have been desired in medical practice. For example, anti IL-5 neutralizing antibody which has been an antibody that neutralizes interleukin 5 causing proliferation and differentiation of eosinophil precursors and extension of survival of mature eosinophils, low-molecular inhibitors of cosinophil-specific adhesion factor Very Late Antigen 4 (VLA-4), and low-molecular antagonists to eosinophil-specific chemokine eotaxin receptor CCR3 causing eosinophil migration have been considered for trying to develop any drugs suppressing eosinophilic inflammations, but these have not been an alternative for steroid drugs.

On the other hand, it has been known that L-threo-3-(3,4-dihydroxyphenyl)-N-[3-(4-fluorophenyl)propyl]serine pyrrolidine amide has an inhibitory effect on eosinophil migration (Patent Document 1). A specific binding site of L-threo-3-(3,4-dihydroxyphenyl)-N-[3-(4-fluorophenyl)propyl]serine pyrrolidine amide *in vivo* has been a receptor-like membrane protein, which has been also referred to as SMBS protein (SMBP) (Patent Document 1).

Accordingly, it is possible to treat allergy diseases including asthma, chronic obstructive pulmonary diseases when eosinophil migration may be inhibited by binding to the SMBS protein.

It has been known that some low-molecular compounds have been useful as a therapeutic agent for allergy diseases

including asthma by binding to the SMBS protein (Patent Documents 2, 3, 4).

[Patent Document 1] WO 98/26065 pamphlet

[Patent Document 2] WO 02/002542 pamphlet
[Patent Document 3] WO 2003/057693 pamphlet
[Patent Document 4] JP-A-2005-206515

DISCLOSURE OF INVENTION

Problem to be Resolved by the Invention

The problem to be resolved by the present invention is to provide an effective compound as a therapeutic agent for various inflammations by inhibiting infiltration of leukocytes including eosinophils, lymphocytes, etc., which may be high safety in long-term administration, and a medicament comprising the same.

Means of Solving the Problem

Since it has been believed that drugs for treating asthma, etc. are administered for long periods, the use of high safety drugs has been especially desired. It has been shown that compounds disclosed in Patent Document 2 have inhibitory effects on rat steroid hormone synthesis in significantly high compound concentrations.
In order to solve the problem, the present inventors have found compounds which are effective as a therapeutic agent for various inflammations by inhibiting infiltration of leukocytes including eosinophils, lymphocytes, etc. via binding to SMBS and significantly reduce inhibitory effects on the steroid hormone synthesis by screening using pharmacological tests and assessment tests for rat steroid hormone synthesis inhibition disclosed in Patent Document 2 in compounds with a structural feature that phenyl group substituted by morpholinoalkyl, morpholinocarbonyl, dialkylaminoalkyl or dialkylamino carbonyl is attached on 4-position of thiazoline, and have achieved the present invention.

The present invention of the present application is as follows:

[1] A 5-membered ring compound of formula (1):

[Chemical Formula 1]

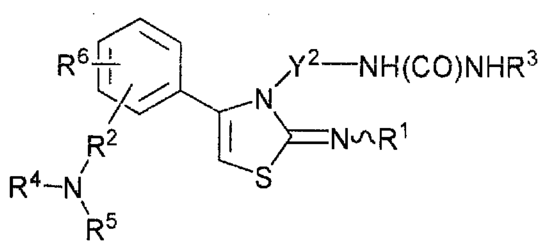

wherein $R^1$ is phenyl optionally substituted by a halogen atom, $C_1$-$C_3$ alkyl or $C_1$-$C_3$ alkoxy;
$R^2$ is $C_1$-$C_3$ alkylene optionally substituted by $C_1$-$C_3$ alkyl, or carbonyl;
$R^3$ is $C_1$-$C_3$ alkyl;
$R^4$ and $R^5$ are each independently hydrogen atom or $C_1$-$C_3$ alkyl; or -N($R^4$)$R^5$ may be morpholino optionally substituted by $C_1$-$C_3$ alkyl;
$Y^2$ is $C_2$-$C_4$ alkylene;
$R^6$ is hydrogen atom, a halogen atom, $C_1$-$C_3$ alkyl or $C_1$-$C_3$ alkoxy; or a pharmaceutically acceptable salt thereof.

[2] The 5-membered ring compound of [1], wherein -N($R^4$)$R^5$ is morpholino optionally substituted by $C_1$-$C_3$ alkyl, or a pharmaceutically acceptable salt thereof.
[3] The 5-membered ring compound of [1], wherein -N($R^4$)$R^5$ is morpholino, or a pharmaceutically acceptable salt thereof.
[4] The 5-membered ring compound of [1], wherein $R^2$ is methylene, or a pharmaceutically acceptable salt thereof.
[5] The 5-membered ring compound of [1], wherein $R^1$ is phenyl optionally substituted by a halogen atom, or a

pharmaceutically acceptable salt thereof.

[6] The 5-membered ring compound of [1] to [5], wherein $R^3$ is methyl or ethyl, and $Y^2$ is $C_2$-$C_3$ alkylene, or a pharmaceutically acceptable salt thereof.

[7] The 5-membered ring compound of any one of [1] to [6], wherein the wavy line represents (Z)-coordination, or a pharmaceutically acceptable salt thereof.

[8] A therapeutic agent for inflammations, comprising the 5-membered ring compound of any one of [1] to [7] or a pharmaceutically acceptable salt thereof.

[9] A therapeutic agent for autoimmune inflammations or allergic inflammations, comprising the 5-membered ring compound of any one of [1] to [7] or a pharmaceutically acceptable salt thereof.

[10] A therapeutic agent for chronic obstructive pulmonary diseases, comprising the 5-membered ring compound of any one of [1] to [7] or a pharmaceutically acceptable salt thereof.

[11] A therapeutic agent for asthma, comprising the 5-membered ring compound of any one of [1] to [7] or a pharmaceutically acceptable salt thereof.

[12] A therapeutic agent for rhinitis, comprising the 5-membered ring compound of any one of [1] to [7] or a pharmaceutically acceptable salt thereof.

[13] A method for treating inflammations, comprising administering the 5-membered ring compound of any one of [1] to [7] or a pharmaceutically acceptable salt thereof to a patient in need thereof.

[14] Use of the 5-membered ring compound of any one of [1] to [7] or a pharmaceutically acceptable salt thereof in the manufacture of a therapeutic agent for inflammations.

Effect of Invention

The 5-membered ring compound or a pharmaceutically acceptable salt thereof of the present invention inhibits infiltration of leukocytes including eosinophils, lymphocytes, etc., and therefore, it has been possible to provide a therapeutic agent for various inflammations.

BEST MODE FOR CARRYING OUT THE INVENTION

Each substituent has the following meaning throughout the specification.
The "$C_1$-$C_3$ alkyl" includes straight- or branched-chain $C_1$-$C_3$ alkyl, particularly methyl, ethyl, n-propyl, 2-propyl. A preferable one includes methyl, ethyl.

The "$C_1$-$C_3$ alkoxy" includes straight- or branched-chain $C_1$-$C_3$ alkoxy, particularly methoxy, ethoxy, n-propoxy, 2-propoxy. A preferable one includes methoxy, ethoxy.

The "halogen atom" includes fluorine, chlorine, bromine, iodine, preferably fluorine, chlorine, bromine, more preferably fluorine, chlorine.

The "$C_2$-$C_4$ alkylene" includes straight- or branched-chain $C_2$-$C_4$ alkylene, particularly ethylene, trimethylene, tetramethylene, methylethylene, 2-methyltrimethylene, etc.. A preferable one includes ethylene, trimethylene.
When $R^1$ is substituted by substituents, the substituents of $R^1$ are the same or different and the number of the substituents includes 1 to 3.

The 5-membered ring compound of formula (1) of the present invention may be a pharmaceutically acceptable salt. The pharmaceutically acceptable salt includes an acid addition salt and a base addition salt. The acid addition salt includes an inorganic acid salt such as hydrochloride, hydrobromide, sulfate, and an organic acid salt such as citrate, oxalate, malate, tartrate, fumarate, maleate, etc..
The compound encompassed in the present invention may have asymmetric centers or any substituents with asymmetric carbons, and may have any optical isomers and geometric isomers. The present invention includes a mixture of each isomer and an isolated one. The present invention also includes a solvate including hydrate of the 5-membered ring compound or a pharmaceutically acceptable salt thereof.

The 5-membered ring compound of formula (1) of the present invention may be prepared according to the following methods and modifications thereof.

Preparation 1

Compound (1) is prepared by the following method.

[Chemical Formula 2]

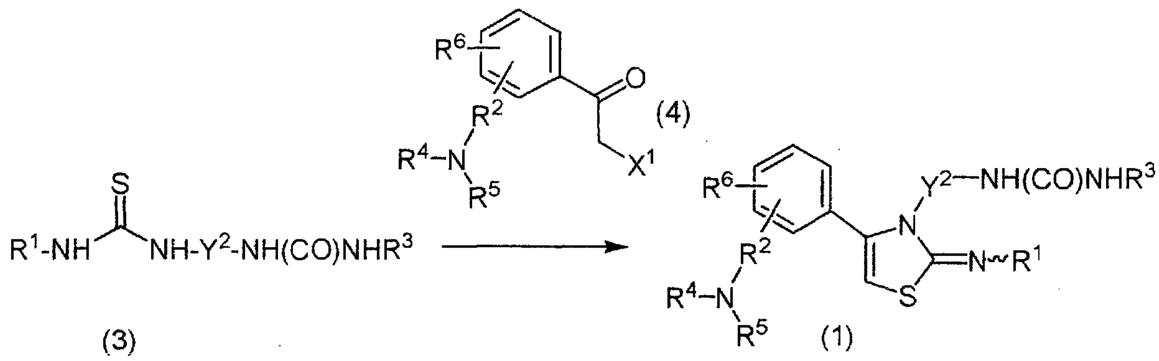

(4)

(3)

(1)

[In the formula, $R^1$, $R^2$, $R^3$, $R^4$, $R^5$, $R^6$ and $Y^2$ have the same meanings as defined above. $X^1$ is a halogen atom such as chlorine, bromine.]
Thiourea compound (3) is reacted with α-haloketone compound (4) in a solvent in the presence or absence of a base to give compound (1). The solvent includes an alcohol solvent such as methanol, ethanol, 2-propanol, an ether solvent such as diethylether, tetrahydrofuran (THF), a halogenated hydrocarbon solvent such as dichloromethane, dichloroethane, chloroform, an aprotic solvent such as dimethylformamide, an aromatic solvent such as toluene, etc.. The base includes an organic amine such as triethylamine, pyridine, 4-dimethylaminopyridine, an inorganic base such as potassium carbonate, sodium carbonate, etc.. The reaction temperature is selected in the range of room temperature to a boiling point of a solvent.

Preparation 2

Compound (1) is also prepared according to the following method. The preparation is useful when a protecting group is needed in the introduction of $R^3$. A conventional protecting group of amino group may be used as the protecting group, and the following preparations are illustrated by using 2-methyl-2-propyloxycarbonyl, which is referred to as Boc hereinafter, as the protecting group.
[Chemical Formula 3]

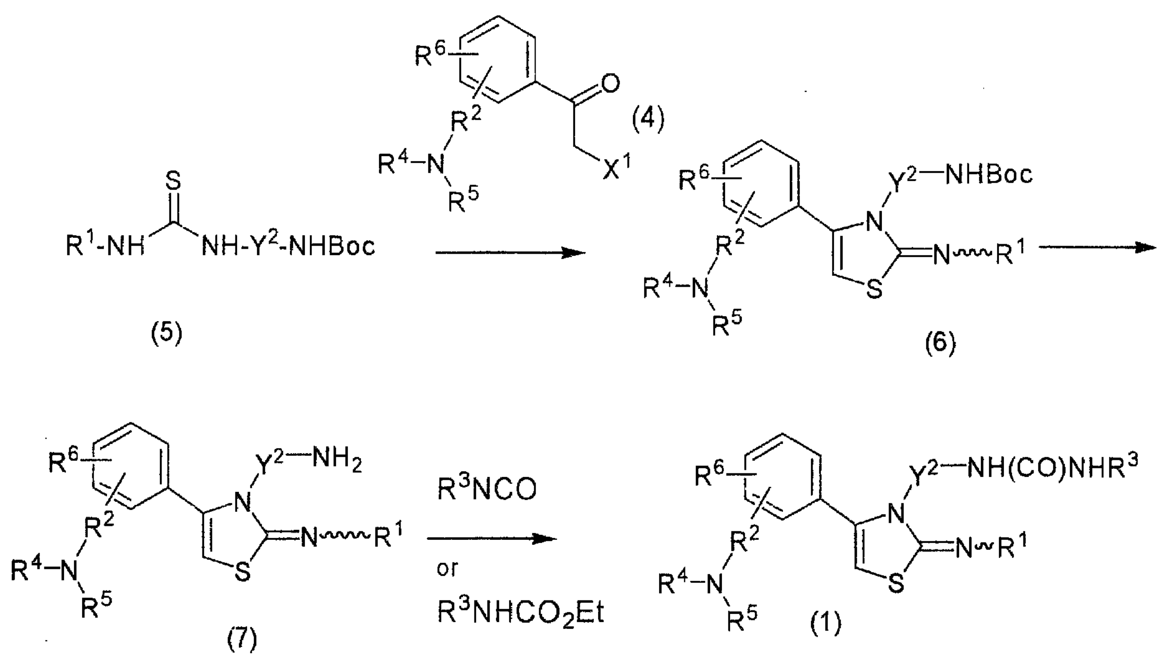


(4)

(5)

(6)

(7)

(1)

[In formula, $R^1$, $R^2$, $R^3$, $R^4$, $R^5$, $R^6$ and $Y^2$ have the same meanings as defined above. $X^1$ is a halogen atom such as

chlorine, bromine.]

Thiourea compound (5) is reacted with α-haloketone compound (4) in a solvent in the presence or absence of a base to give compound (6). The solvent includes an alcohol solvent such as methanol, ethanol, 2-propanol, an ether solvent such as diethylether, THF, a halogenated hydrocarbon solvent such as dichloromethane, dichloroethane, chloroform, an aprotic solvent such as dimethylformamide, an aromatic solvent such as toluene, etc.. The base includes an organic amine such as triethylamine, pyridine, 4-dimethylaminopyridine, and an inorganic base such as potassium carbonate, sodium carbonate. The reaction temperature is selected in the range of room temperature to a boiling point of a solvent.

Then, compound (6) is deprotected in the presence of an acid in a solvent to give compound (7). The acid includes an inorganic acid such as hydrochloric acid, and an organic acid such as trifluoroacetic acid. The solvent includes an ether solvent such as diethylether, THF, dioxane, a halogenated hydrocarbon solvent such as dichloromethane, dichloroethane, chloroform, etc.. The reaction temperature is selected in the range of about 0°C to a boiling point of a solvent.

Compound (7) is reacted with a compound such as a corresponding isocyanate or carbamic acid ester in the presence or absence of a base in a solvent to give compound (1). A concrete example of the compound includes alkyl isocyanate, ethyl alkyl carbamate, etc.. The solvent includes an ether solvent such as diethylether, THF, a halogenated hydrocarbon solvent such as dichloromethane, dichloroethane, chloroform, an aprotic solvent such as dimethylformamide, an aromatic solvent such as toluene, etc.. The base includes an organic amine such as triethylamine, pyridine, 4-dimethylaminopyridine, an inorganic base such as potassium carbonate, sodium carbonate, etc.. The reaction temperature is selected in the range of room temperature to a boiling point of a solvent.

Preparation 3

Compound (1) is also prepared according to the following method.

[Chemical Formula 4]

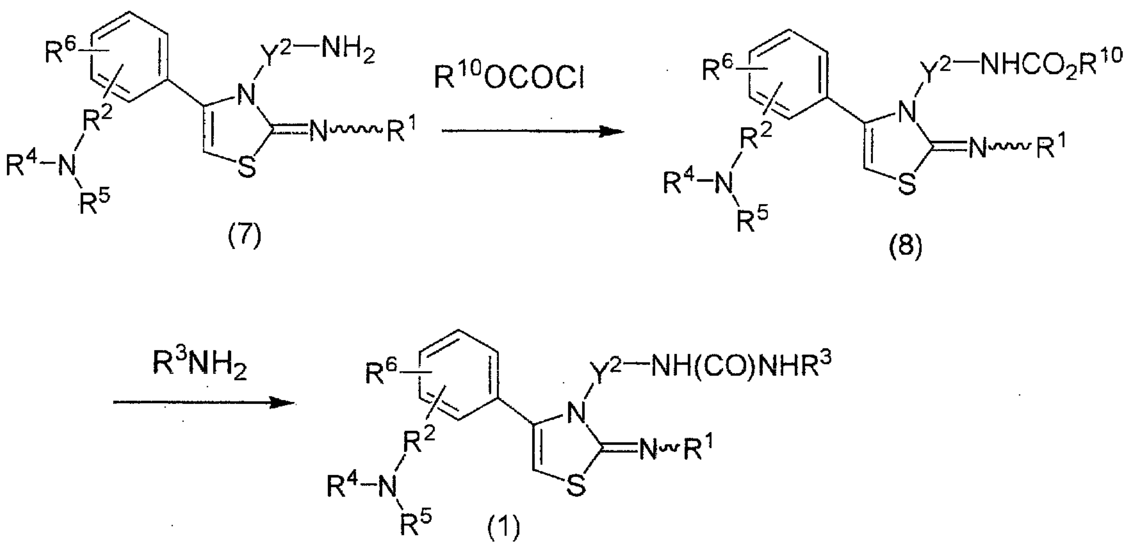


(7) (8) (1)

[In formula, $R^1$, $R^2$, $R^3$, $R^4$, $R^5$, $R^6$ and $Y^2$ have the same meanings as defined above. $R^{10}$ is alkyl, or phenyl substituted by a halogen atom or nitro.]

Compound (7) is reacted with chloroformate (e.g., $R^{10}OCOCl$ wherein $R^{10}$ is the same as defined above) in a solvent in the presence or absence of a base to give compound (8). The solvent includes an ether solvent such as diethylether, THF, a halogenated hydrocarbon solvent such as dichloromethane, dichloroethane, chloroform, an aprotic solvent such as dimethylformamide, an aromatic solvent such as toluene, etc.. The base includes an organic amine such as triethylamine, pyridine, 4-dimethylaminopyridine, and an inorganic base such as potassium carbonate, sodium carbonate. The reaction temperature is selected in the range of room temperature to a boiling point of a solvent.

Compound (8) is reacted with an amine of $R^3NH_2$ in a solvent in the presence or absence of a base to give compound (1). The solvent includes an ether solvent such as diethylether, THF, dioxane, a halogenated hydrocarbon solvent such as dichloromethane, dichloroethane, chloroform, etc.. The base includes the base as described above. The reaction temperature is selected in the range of about 0°C to a boiling point of a solvent.

Starting materials used in the above Preparations I to 3 are prepared according to the following methods.

[Chemical Formula 5]

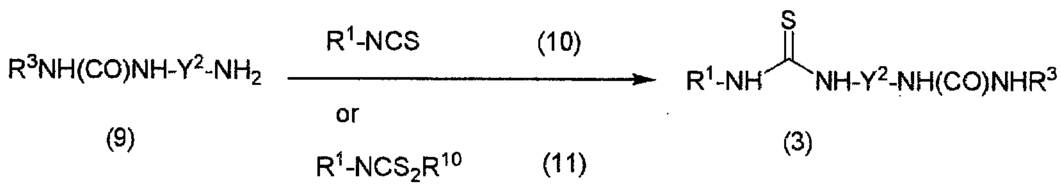


(9) (3)

[In formula, $R^1$, $R^2$, $R^3$, $R^{10}$ and $Y^2$ have the same meanings as defined above.]

Amine compound (12) may be reacted with isocyanate compound (13) or dithiocarbamic acid ester (14) in a solvent to give thiourea compound (3). The solvent includes an alcohol solvent such as methanol, ethanol, 2-propanol, an ether solvent such as diethylether, THF, a halogenated hydrocarbon solvent such as dichloromethane, dichloroethane, chloroform, an aprotic solvent such as dimethylformamide, an aromatic solvent such as toluene, etc.. The reaction temperature is selected in the range of room temperature to a boiling point of a solvent.

[Chemical Formula 6]

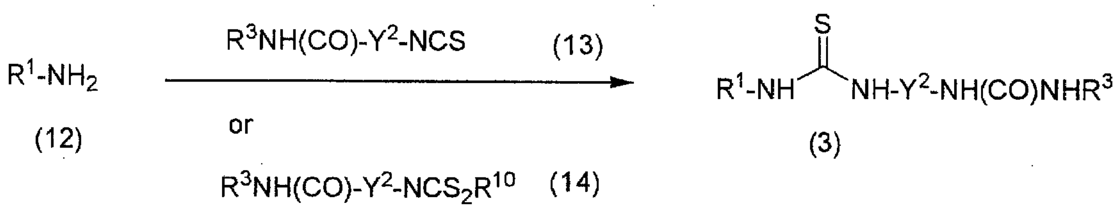


(12) (3)

[In formula, $R^1$, $R^2$, $R^5$, $R^{10}$ and $Y^2$ have the same meanings as defined above.]

Amine compound (12) may be reacted with isocyanate compound (13) or dithiocarbamic acid ester (14) in a solvent to give thiourea compound (3). The solvent includes an alcohol solvent such as methanol, ethanol, 2-propanol, an ether solvent such as diethylether, THF, a halogenated hydrocarbon solvent such as dichloromethane, dichloroethane, chloroform, an aprotic solvent such as dimethylformamide, an aromatic solvent such as toluene, etc.. The reaction temperature is selected in the range of room temperature to a boiling point of a solvent.

Thiourea compound (5) which is protected by 2-methyl-2-propyloxycarbonyl, etc. is obtained in the following method.

[Chemical Formula 7]

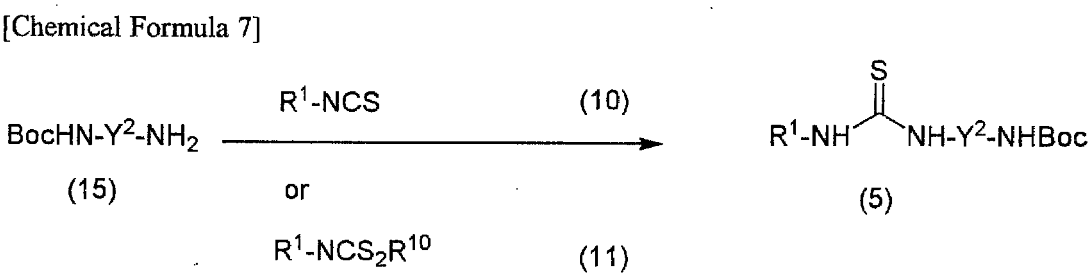


(15) (5)

[In formula, $R^1$, $R^{10}$, $Y^2$ and Boc have the same meanings as defined above.]

Amine compound (15) may be reached with isocyanate compound (10) or dithiocarbamic acid ester (11) in a solvent to give thiourea compound (5). The solvent includes an alcohol solvent such as methanol, ethanol, 2-propanol, an ether solvent such as diethylether, THF, a halogenated hydrocarbon solvent such as dichloromethane, dichloroethane, chloroform, an aprotic solvent such as dimethylformamide, an aromatic solvent such as toluene, etc.. The reaction

temperature is selected in the range of room temperature to a boiling point of a solvent.

[Chemical Formula 8]

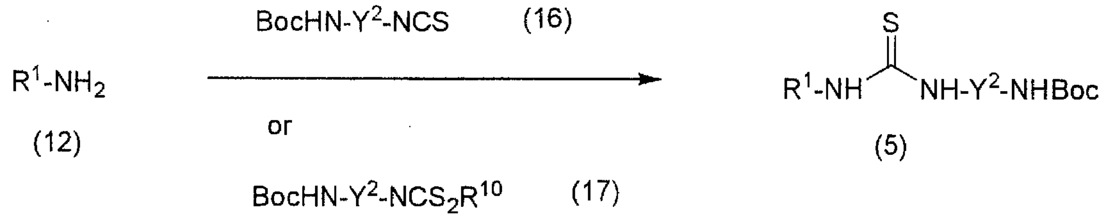


[In formula, $R^1$, $R^{10}$, $Y^2$ and Boc have the same meanings as defined above.]

Amine compound (12) may be reacted with isocyanate compound (16) or dithiocarbamic acid ester (17) in a solvent to give thiourea compound (5). The solvent includes an alcohol solvent such as methanol, ethanol, 2-propanol, an ether solvent such as diethylether, THF, a halogenated hydrocarbon solvent such as dichloromethane, dichloroethane, chloroform, an aprotic solvent such as dimethylformamide, an aromatic solvent such as toluene, etc.. The reaction temperature is selected in the range of room temperature to a boiling point of a solvent.

Isothiocyanate compounds (10), (13) and (16) are commercially available, or may be synthesized from corresponding amino compounds according to the methods of Synlett. 1997, 773-774, J. Org. Chem., 1997, 62, 4539-4540, or J. Med. Chem., 1984, 27, 1570-1574, for example, and may be also synthesized from corresponding carboxylic acid compounds according to the methods of Synth. Commun. 1997, 27, 751-756, or Indian, J. Chem., 1998, 1153-1156, for example.

Dithiocarbamic acid ester compounds (11), (14) and (17) are commercially available, or may be synthesized from corresponding amino compounds according to the methods of J. Chem. Soc. 1956, 1644-1649, or Syn. Commun. 1984, 537-546, for example.

$\alpha$-Haloketone compound (4) is commercially available, or may be synthesized from a corresponding keton compound according to the method of J. Med. Chem. 1987, 1497-1502, Tetrahedoron Lett. 1998, 4987-4990, or Acta Chim. Scand. 1986, B40, 700-702, for example.

The 5-membered ring compound (1) or an intermediate for preparing the same of the present invention may be purified in a conventional manner. The 5-membered ring compound (1) or an intermediate for preparing the same of the present invention with several isomers may be also purified in a similar manner. For example, the compound may be purified by column chromatography, recrystallization, etc.. A solvent for recrystallization includes an alcoholic solvent such as methanol, ethanol, 2-propanol, an ether solvent such as diethylether, an ester solvent such as ethyl acetate, an aromatic hydrocarbon solvent such as toluene, a ketone solvent such as acetone, a hydrocarbon solvent such as hexane, or a mixed solvent thereof, etc..

A method for obtaining a pure optical isomer includes an optical resolution method. The optical resolution method includes a method for treating the present compound or an intermediate thereof which has a basic substituent such as amino group with an optically active acid (e.g., monocarboxylic acids such as mandelic acid, N-benzyloxyalanine, lactic acid, dicarboxylic acids such as tartaric acid, O-diisopropylidenetartaric acid, malic acid, sulfonic acids such as camphersulfonic acid, bromocamphersulfonic acid) in an inactive solvent (e.g., an alcoholic solvent such as methanol, ethanol, 2-propanol, an ether solvent such as diethylether, an ester solvent such as ethyl acetate, an aromatic hydrocarbon solvent such as toluene, acetonitrile, and a mixed solvent thereof) to form a salt. A method for treating the present compound or an intermediate thereof which has an acidic substituent such as carboxyl group with an optically active amine (e.g., organic amines such as $\alpha$-phenethylamine, kinin, quinidine, cinchonidine, cinchonine, strychnine) to form a salt may be adopted. The reaction temperature in forming a salt in the optical resolution method includes the range of room temperature to a boiling point of a solvent. In order to improve the optical purity, the reaction temperature is preferably raised to around a boiling point of a solvent once. The yields may be optionally improved by cooling before the filtration of the precipitated salt, if necessary. The usage of the optically active acid or amine is appropriately in the range of about 0.5 to about 2.0 equivalents, preferably around 1 equivalent, to the substrate. The resulting crystal may be optionally recrystallized in an inactive solvent (e.g., an alcoholic solvent such as methanol, ethanol, 2-propanol, an ether solvent such as diethylether, an ester solvent such as ethyl acetate, an aromatic hydrocarbon solvent such as toluene, acetonitrile, and a mixed solvent thereof) to give an optically active salt in high yields. The resulting salt may be also optionally treated with an acid or a base in a conventional manner to give a free form.

The 5-membered ring compound of formula (1) or a pharmaceutically acceptable salt thereof, or a prodrug thereof of the present invention is useful as a medicament, especially with an inhibitory effect on infiltration of leukocytes

including eosinophils, lymphocytes, etc.. Due to the effect, the present invention is useful as a therapeutic agent for autoimmune inflammations, allergic inflammations, acute inflammations, other cell infiltrative inflammatory diseases, etc.. The autoimmune inflammations include rheumatism, multiple sclerosis, inflammatory bowel diseases, type I diabetes, etc.. The allergic inflammations include bronchial asthma, inflammatory bowel diseases, allergic rhinitis, atopic dermatitis, hives, allergic conjunctivitis, etc.. The present 5-membered ring compound is especially useful for late asthma in bronchial asthma. The acute inflammations include inflammatory lung diseases, etc.. The other inflammatory diseases include eosinophilia, eosinophilic angiitis, eosinophilic granuloma, transplantation rejection, tumor metastasis, etc.. The present compound used as anti-inflammatory drugs may be administered in combination with steroid drugs as a therapeutic agent for inflammatory diseases, and the combination use may enhance the therapeutic effects of the present compound and allow for the reduction or elimination of steroid drugs with strong adverse effects. The present compound used as a therapeutic agent for allergy diseases may be administered in combination with an antiallergic agent (including an inhibitor of liberation of chemical messengers, antihistamine agent, antileukotriene agent, antithromboxane agent, etc.), and in bronchial asthma, a bronchodilator agent (including xanthine preparation including theophylline, β-stimulant), anticholinergic agents, steroid drugs. The present compound used as a therapeutic agent for autoimmune diseases including rheumatism may be administered in combination with nonsteroidal anti-inflammatory drugs including cyclooxygenase (COX) inhibitors.

The present 5-membered ring compound or a pharmaceutically acceptable salt thereof, or a prodrug thereof may be administered orally or parenterally. The oral administration may be carried out in the conventional dosage form. The parenteral administration may be carried out in the form of topical administration, injection, transdermal administration, transnasal administration, etc.. A preparation for oral or rectal administration includes a capsule, a tablet, a pill, a powder, a cachet, a suppository, a solution, etc.. The injection includes a sterile solution or a suspension, etc.. The topical preparation includes a cream, an ointment, a lotion, a transdermal preparation (including a conventional patch, matrix), etc..

The above dosage form is formulated using a pharmaceutically acceptable excipient and additive in a conventional manner. The pharmaceutically acceptable excipient and additive include a carrier, a binder, a perfume, a buffer, a thickener, a colorant, a stabilizer, an emulsifier, a dispersant, a suspending agent, a preservative agent, etc..

The pharmaceutically acceptable carrier includes magnesium carbonate, magnesium stearate, talc, sugar, lactose, pectin, dextrin, starch, gelatin, tragacanth, methylcellulose, sodium carboxymethyl cellulose, low-melting wax, cocoa butter, etc..

The capsule may be formulated by setting the present compound in a capsule together with a pharmaceutically acceptable carrier. The present compound may be set in a capsule by mixing with a pharmaceutically acceptable excipient, or without any excipient. The cachet may be also formulated in a similar manner.

The powder is formulated together with a pharmaceutically acceptable powder base. The base includes talc, lactose, starch, etc.. The drop may be formulated together with aqueous or nonaqueous base and one or more of a pharmaceutically acceptable diffusing agent, suspending agent, solubilizer, etc..

The injectable solution includes a solution, a suspension, an emulsion, for example aqueous solution, water-propylene glycol solution, etc.. The solution may contain water, and may be also prepared in the form of a solution of polyethylene glycol or/and propylene glycol. The appropriate solution for oral administration may be prepared by adding the present compound to water together with a colorant, a perfume, a stabilizing agent, a sweetening agent, a solubilizer, a thickener, etc., if necessary. The appropriate solution for oral administration may be also prepared by adding the present compound to water together with a dispersant to thicken. The thickener includes a pharmaccutically acceptable natural or synthetic gum, resin, methylcellulose, sodium carboxymethyl cellulose, or known suspending agent.

The topical formulation includes the solution, and a cream, an aerosol, a spray, a powder, a lotion, an ointment, etc.. The topical formulation may be prepared by mixing the present compound with a conventional pharmaceutically acceptable diluent and carrier. The ointment and cream may be formulated by adding a thickener and/or a gelator to an aqueous or oily base. The base includes water, liquid paraffin, vegetable oil (including peanut oil, castor oil). The thickener includes soft paraffin, aluminum stearate, cetostearyl alcohol, propyleneglycol, polyethylene glycol, lanolin, hydrogenated lanolin, bees wax, etc..

For the lotion, one or more of a pharmaceutically acceptable stabilizer, suspending agent, emulsifier, diffusing agent, thickener, colorant, perfume, etc. may be added to an aqueous or oily base.

The topical formulation may optionally contain an antiseptic, a cell-proliferation preventive agent including methyl hydroxybenzoate, propyl hydroxybenzoate, chlorocresol, benzalkonium chloride.

The present compound may be also nasally administered in the form of a solution spray, a powder or a drop formulation.

The dosage amounts and the number of doses may vary depending on conditions, ages, weights, dosage forms, and may be orally administered in the range of about I to about 1000 mg, preferably about 2 to about 500 mg, particularly preferably about 5 to about 100 mg, once or in several divided doses per day to an adult. The administration by injection may be in the range of about 0.1 to about 300 mg, preferably about 1 to about 200 mg, once or in several

divided doses.

EXAMPLES

Example 1

The present invention is illustrated with Examples more specifically, but is not limited thereto. Reference Example 1

4-[4-(Morpholin-4-ylcarbonyl)benzyl]morpholine

[Chemical Formula 9]

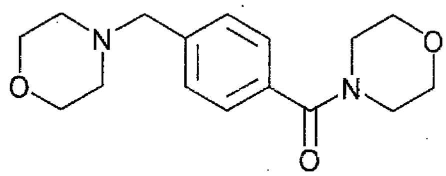

To a solution of 4-(hydroxylmethyl)benzoic acid (9.13 g) in methylene chloride (70 ml) solution were added thionyl chloride (28.6 g) and N,N-dimethylformamide (1 ml), and the mixture was heated to reflux for three hours. The reaction mixture was evaporated under reduced pressure and azeotroped with toluene (250 ml) twice, and then the resultant was dissolved in methylene chloride (70 ml). To the mixture were added morpholine (15.7 g) and triethylamine (18.2 g) at 0˚C, and then the mixture was stirred at room temperature for 12 hours. To the reaction mixture was added aqueous saturated sodium bicarbonate solution (250 ml), and then the mixture was extracted with chloroform. The organic layer was dried over sodium sulfate, and then the solvent was evaporated under reduced pressure. The residue was purified by silica gel column chromatography [1.5% methanol-chloroform] to give the titled compound (13.7 g) as a pale yellow crystal.
$^1$H-NMR($CDCl_3$): δ 2.44(4H, m), 3.41-3.77(12H, m), 7.37(4H, m)

Reference Example 2

1-[4-(Morpholin-4-ylmethyl)phenyl]ethanone

[Chemical Formula 10]

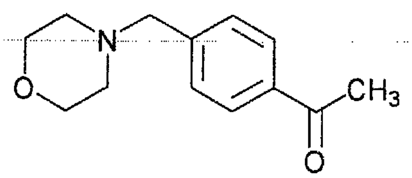

To a solution of 4-[4-(morpholin-4-ylcarbonyl)benzyl]morpholine (13.7 g) obtained in Reference Example 1 in toluene (150 ml) was added 0.87M methyl Grignard reagent-tetrahydrofuran solution (65 ml), and the mixture was stirred under nitrogen at 70˚C for three hours. To the reaction mixture was added aqueous saturated ammonium chloride solution (70 ml), and the mixture was extracted with ethyl acetate. The organic layer was washed with a saturated saline, and then dried over sodium sulfate and the solvent was evaporated under reduced pressure. The residue was purified by silica gel column chromatography [chloroform] to give the titled compound (7.4 g) as a pale yellow crystal.
$^1$H-NMR($CDCl_3$): δ 2.45(4H, m), 2.60 (3H, s), 3.72(4H, m), 7.44(2H, d, J = 8.1), 7.92(2H, d, J = 8.1)

Reference Example 3

(2R,6S)-4-(4-{[(2R,6S)-2,6-Dimethylmopholin-4-yl]carbonyl}benzyl)-2,6-dimethylmorpholine

[Chemical Formula 11]

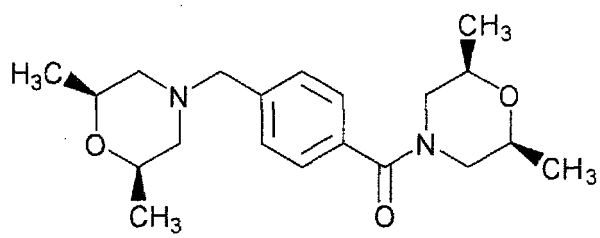

4-(Hydroxylmethyl)benzoic acid (5.33 g) and cis-2,6-dimethylmorpholine (10.1 g) were used in a similar manner to Reference Example 1 to give the titled compound (12.0 g) as a pale yellow crystal. $^1$H-NMR($CDCl_3$): δ 1.08-1.28(12H, m), 1.76(2H, m), 2.69(4H, m), 3.54(2H, s), 3.56-3.71(5H, m), 4.57(1H, brd), 7.36(4H, m)

Reference Example 4

1-(4-{[(2R,6S)-2,6-Dimethylmorpholin-4-yl]methyl}phenyl)ethanone

[Chemical Formula 12]

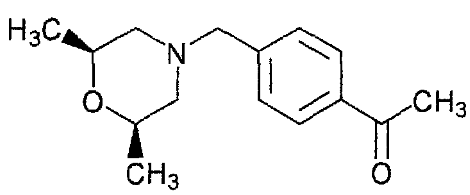

(2R,6S)-4-(4-{[(2R,6S)-2,6-Dimethylmorpholin-4-yl]carbonyl}benzyl)-2,6-dimethylmorpholine (4.25 g) obtained in Reference Example 3 was treated in a similar manner to Reference Example 2 to give the titled compound (2.87 g) as a pale yellow crystal.
$^1$H-NMR($CDCl_3$): δ 1.13(6H, d J=6.0), 1.77(2H, t), 2.60(3H, s), 2.67(2H, td), 3.60(3H, s), 3.69(2H, m), 7.43(2H, d, J = 8.3Hz), 7.92(2H, d, J = 8.3Hz)

Example 1

N-{2-[(2Z)-2-[(4-Fluoro-3-methylphenyl)imino]-4-[4-(morpholin-4-ylmethyl)phenyl]-1,3-thiazol-3(2H)-yl]ethyl}-N'-methylurea

[Chemical Formula 13]

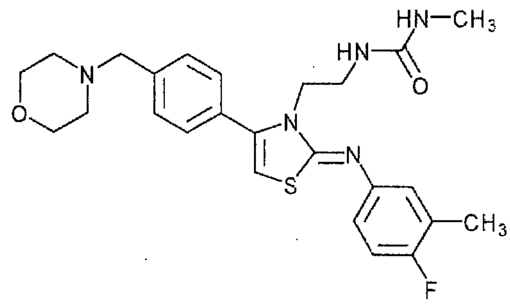

To a solution of 1-[4-(morpholin-4-ylmethyl)phenyl]ethanone (1.75 g) obtained in Reference Example 2 in chloroform (40 ml) was added dropwise bromine (1.92 g)-chloroform (12 ml) at room temperature, and the mixture was stirred for 1 hour at the same temperature. The solvent was evaporated under reduced pressure, and then thereto were added N-[2-({[(4-fluoro-3-methylphenyl)amino]carbonothioyl}amino)ethyl]-N'-methylurea (1.77 g) and ethanol (20 ml). The mixture was stirred at 70˚C for 2 hours. Then, the mixture was stirred at room temperature overnight, and then the precipitated white solid was filtered and washed with ethanol (5 ml) three times. The resulting solid was dried under reduced pressure, and then thereto was added aqueous saturated sodium bicarbonate solution. The mixture was extracted with chloroform. The organic layer was dried over sodium sulfate, and then the solvent was evaporated under reduced pressure. Then, to the residue was added ethyl acetate (30 ml), and the mixture was stirred. The precipitated crystal was filtered and dried under reduced pressure to give the titled compound (1.25 g) as a colorless crystal.
Melting Point: 142-145˚C
$^1$H-NMR($CDCl_3$): δ 2.28(3H, s), 2.49(4H, m), 2.66(3H, d, J=4.8), 3.42(2H, q, J=6.4), 3.55(2H, s), 3.74(4H, m), 3,87(2H, t, J=6.4), 5.78(1H, s), 6.85-6.92(2H, m), 6.99(1H, t J=8.9), 7.31(2H, d, J=8.1), 7.43(2H, d, J=8.1)

Examples 2 to 17

The acetophenones obtained in Reference Examples 2 and 4 were reacted with various thioureas in a similar manner to Example I to give compounds shown in Table 1.
[Table 1]

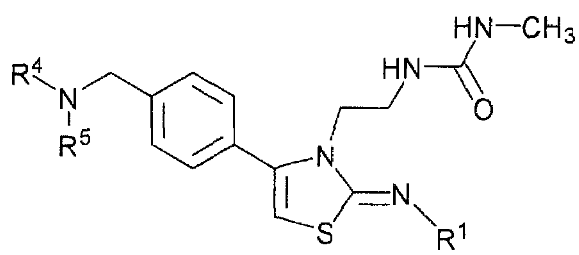

| Example | $R^1$ | $R^4(R^5)N$ | Yields (%) | Melting Point (˚C) |
|---|---|---|---|---|
| 2 | 3-fluorophenyl | morpholino | 43 | 145-148 |
| 3 | 2,5-difluorophenyl | morpholino | 22 | 143-144 |
| 4 | 2,4-difluorophenyl | morpholino | 58 | 156-160 |
| 5 | 3,4-difluorophenyl | morpholino | 36 | 143-147 |
| 6 | 3,5-difluorophenyl | morpholino | 46 | 145-151 |
| 7 | 2,3-difluorophenyl | morpholino | 35 | 160-162 |
| 8 | 4-fluoro-2-methylphenyl | morpholino | 41 | 178-183 |
| 9 | 5-fluoro-2-methylphenyl | morpholino | 28 | 143-146 |

(continued)

| Example | $R^1$ | $R^4(R^5)N$ | Yields (%) | Melting Point (˚C) |
|---|---|---|---|---|
| 10 | 3-fluoro-2-methylphenyl | morpholino | 46 | 161-164 |
| 11 | 3-fluoro-4-methylphenyl | morpholino | 25 | 135-137 |
| 12 | 2,3-difluorophenyl | cis-2,6-dimethylmorpholino | 53 | 135-138 |
| 13 | 2,4-difluorophenyl | cis-2,6-dimethylmorpholino | 28 | 135-139 |
| 14 | 3-fluoro-2-methylphenyl | cis-2,6-dimethylmorpholino | 20 | 113-115 |
| 15 | 3-fluoro-4-methylphenyl | cis-2,6-dimethylmorpholino | 32 | 136-140 |
| 16 | 4-fluoro-2-methylphenyl | cis-2,6-dimethylmorpholino | 39 | 149-152 |
| 17 | 4-fluoro-3-methylphenyl | cis-2,6-dimethylmorpholino | 32 | 143-145 |

Reference Example 5

Methyl 4-cyano-2-methylbenzoate

[Chemical Formula 14]

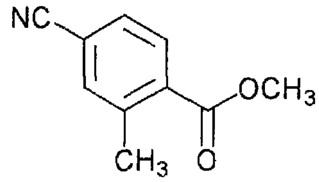

To a solution of methyl 4-bromo-2-methylbenzoate (34.5 g) in 1-methyl-2-pyrrolidinone (210 ml) was added copper (I) cyanide (16.1 g), and the mixture was heated and stirred at 180˚C for 12 hours. After leaving to cool to room temperature, and to the mixture was added dropwise water (250 ml) with stirring. The precipitated solid was filtered, and then washed with water and dried under reduced pressure. Then, the resulting brown solid was extracted with acetone (250 ml x 3) and filtered, and the filtrate was concentrated under reduced pressure to give the titled compound (26.0 g) as a pale yellow oil.

$^1$H-NMR($CDCl_3$): δ 2.62(3H, s), 2.85(3H, s), 7.54(1H, d, J=8.6), 7.55(1H, s), 7.97(1H, d, J=8.6)

Reference Example 6

4-Cyano-N-methoxy-N,2-dimethylbenzamide

[Chemical Formula 15]

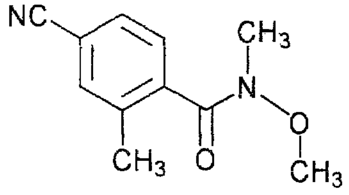

To methyl 4-cyano-2-methylbenzoate (26.0 g) obtained in Reference Example 5 were added methanol (240 ml) and 2N aqueous NaOH solution (120 ml), and the mixture was stirred at room temperature overnight. Then, thereto was added

dropwise 3N aqueous HCl solution (80 ml), and the precipitated solid was filtered and dried under reduced pressure to give 4-cyano-2-methylbenzoic acid as a pale yellow solid (17.0 g).
Then, to a solution of 4-cyano-2-methylbenzoic acid (17.0 g) in DMF (400 ml) were added N,O-dimethylhydroxylamine hydrochloride (13.3 g), 1-(3-dimethylaminopropyl)-3-ethylcarbodiimide hydrochloride (30.2 g), 1-hydroxybenzotriazole monohydrate (24.1 g) and triethylamine (36.6 ml), and the mixture was stirred at room temperature overnight. The reaction mixture was poured into water (500 ml), and extracted with ethyl acetate. The organic layer was washed with water, then a saturated saline, and then dried over sodium sulfate, and the solvent was evaporated under reduced pressure. The residue was purified by silica gel column chromatography [50% hexane-chloroform] to give the titled compound (6.23 g) as a yellow oil.
$^1$H-NMR($CDCl_3$): δ 2.37(3H, s), 3.39(6H, br), 7.37(1H, d, J=8.2), 7.52(2H, m)

Reference Example 7

4-Formyl-N-methoxy-N,2-dimethylbenzamide

[Chemical Formula 16]

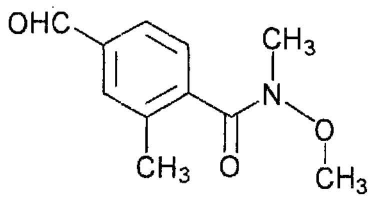

To a solution of 4-cyano-N-methoxy-N,2-dimethylbenzamide (1.40 g) obtained in Reference Example 6 in 80% aqueous formic acid (11 ml) was added platinum (IV) oxide (311 mg), and the mixture was stirred at 60°C for 1 hour. After leaving to cool to room temperature, the mixture was filtered through Celite and the insoluble was filtered off. The filtrate was neutralized by aqueous saturated sodium bicarbonate solution, and then extracted with ethyl acetate. The organic layer was washed with water, then a saturated saline, and then dried over sodium sulfate, and the solvent was evaporated under reduced pressure. The residue was purified by silica gel column chromatography [chloroform] to give the titled compound (1.17g) as a pale yellow oil.
$^1$H-NMR($CDCl_3$): δ 2.42(3H, s), 3.39(6H, br), 7.44(1H, d, J=8.1), 7.74(2H, m), 10.01(1H, s)

Reference Example 8

N-Methoxy-N,2-dimethyl-4-(morpholin-4-ylmethyl)benzamide

[Chemical Formula 17]

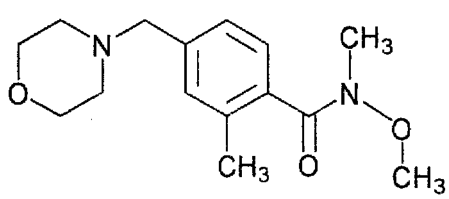

To a solution of 4-formyl-N-methoxy-N,2-dimethylbenzamide (4.23 g) obtained in Reference Example 7 in methylene chloride (150 ml) were added morpholine (1.96 g), sodium triacetoxyborohydride (6.49 g) and acetic acid (1.17 ml) at room temperature overnight. The mixture was neutralized by aqueous saturated sodium bicarbonate solution, and then extracted with chloroform. The organic layer was dried over sodium sulfate, and the solvent was evaporated under reduced pressure. The residue was purified by silica gel column chromatography [1.5% methanol-chloroform] to give the titled compound (5.4 g) as a pale yellow oil.

$^1$H-NMR($CDCl_3$): δ 2.33(3H, s), 2.44(4H, m), 3.30(3H, br), 3.47(2H, s), 3.52(3H, br), 3.71(4H, m),7.17(2H, m), 7.22(1H, d, J=7.7)

Reference Example 9

1-[2-Methyl-4-(morpholin-4-ylmethyl)phenyl]ethanone

[Chemical Formula 18]

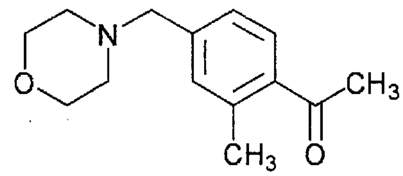

To a solution of N-methoxy-N,2-dimethyl-4-(morpholin-4-ylmethyl)benzamide (1.94 g) obtained in Reference Example 8 in toluene (23 ml) was added 0.87M methyl Grignard reagent-tetrahydrofuran solution (11 ml), and the mixture was stirred under nitrogen at 70°C for three hours. To the reaction mixture was added aqueous saturated ammonium chloride solution (10 ml), and the mixture was extracted with ethyl acetate. The organic layer was washed with a saturated saline, and then dried over sodium sulfate, and the solvent was evaporated under reduced pressure. The residue was purified by silica gel column chromatography [hexane:ethyl acetate = 2:1] to give the titled compound (0.9 g) as a pale yellow oil.
$^1$H-NMR($CDCl_3$) δ 2.45(4H, m), 2.54(3H, s), 2.58(3H, s), 3.49(2H, s), 3.72(4H, m), 7.21(1H, s,), 7.24(1H, d, J=7.9), 7.68 (1H, d, J=7.9)

Example 18

N-{2-[(2Z)-2-[(4-Fluoro-3-methylphenyl)imino]-4-[2-methyl-4-(morpholin-4-ylmethyl)phenyl]-1,3-thiazol-3(2H)-yl] ethyl}-N'-methylurea

[Chemical Formula 19]

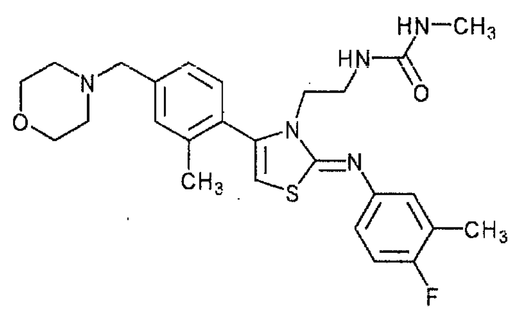

1-[2-Methyl-4-(morpholin-4-ylmethyl)phenyl]ethanone (1.70 g) obtained in Reference Example 9 and N-[2-({[(4-fluoro-3-methylphenyl)amino]carbonothioyl}amino)ethyl]-N'-methylurea (1.56 g) were used in a similar manner to Example 1 to give the titled compound (1.20 g) as a colorless crystal.
Melting Point: 130-135°C
$^1$H-NMR($CDCl_3$): δ 2.24(3H, s), 2.28(3H, s), 2.48(4H, m), 2.66(3H, d, J=4.7), 3.25(2H, br), 3.51(1H, br), 3.52(2H, s), 3.74(4H, m), 3.89(1H, br), 5.72(1H, s), 6.87-6.94(2H, m), 6.99(1H, t, J=8.9), 7.19-7.28(3H, m)

Reference Example 10

Methyl 4-cyano-3-methylbenzoate

[Chemical Formula 20]

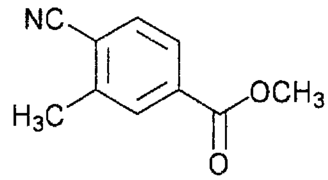

Methyl 4-bromo-3-methylbenzoate (21.3 g) was used in a similar manner to Reference Example 5 to give the titled compound (16.3 g) as a pale yellow oil.
$^1$H-NMR($CDCl_3$): δ 2.61(3H, s), 3.95(3H, s), 7.68(1H, d, J=8.1), 7.93(1H, d, J=8.1), 8.00(1H, s),

Reference Example 11

4-Cyano-N-methoxy-N,3-dimethylbenzamide

[Chemical Formula 21]

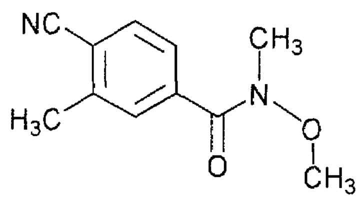

Methyl 4-cyano-3-methylbenzoate (16.3 g) obtained in Reference Example 10 was used in a similar manner to Reference Example 6 to give the titled compound (12.7 g) as a pale yellow crystal. $^1$H-NMR($CDCl_3$): δ 2.59(3H, s), 3.37(3H, s), 3.53(3H, s), 7.55(1H, d, J=7.6), 7.63(2H, m)

Reference Example 12

4-Formyl-N-methoxy-N,3-dimethylbenzamide

[Chemical Formula 22]

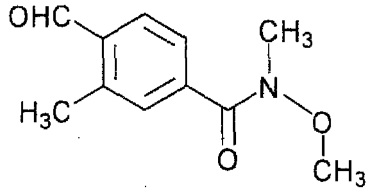

4-Cyano-N-methoxy-N,3-dimethylbenzamide (2.04 g) obtained in Reference Example 11 was treated in a similar manner to Reference Example 7 to give the titled compound (1.02 g) as a pale yellow oil.
$^1$H-NMR($CDCl_3$): δ 2.71(3H, s), 3.37(3H, s), 3.56(3H, s), 7.63(2H, m), 7.84(1H, d, J=7.9), 10.32(1H, s)

Reference Example 13

N-Methoxy-N,3-dimethyl-4-(morpholin-4-ylmethyl)benzamide

[Chemical Formula 23]

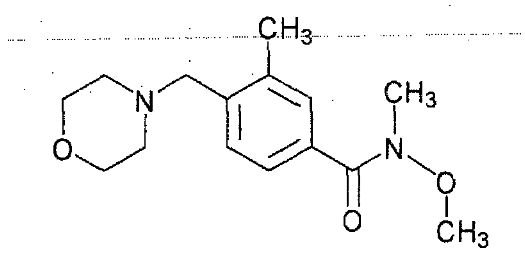

4-Formyl-N-methoxy-N,3-dimethylbenzamide (1.02 g) obtained in Reference Example 12 was treated in a similar manner to Reference Example 8 to give the titled compound (1.30 g) as a pale yellow oil.
$^1$H-NMR($CDCl_3$): δ 2.39(3H, s),2.45(4H, m) 3.35(3H, s),3.47(2H, s) 3.58(3H, s),3.70(4H, m), 7.30(1H, d, J=7.6), 7.43-7.46 (2H, m)

Reference Example 14

1-[3-Methyl-4-(morpholin-4-ylmethyl)phenyl]ethanone

[Chemical Formula 24]

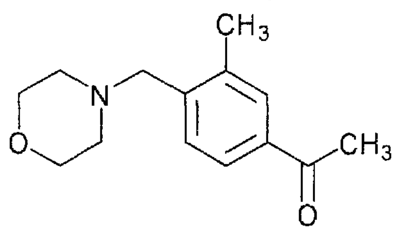

N-Methoxy-N,3-dimethyl-4-(morpholin-4-ylmethyl)benzamide (1.30 g) obtained in Reference Example 13 was treated in a similar manner to Reference Example 9 to give the titled compound (0.81 g) as a pale yellow oil.
$^1$H-NMR($CDCl_3$): δ 2.42(3H, s), 2.45(4H, m), 2.59(3H, s), 3.50(2H, s), 3.70(4H, m), 7.39(1H, d, J=7.8), 7.73-7.76(2H, m)

Example 19

N-{2-[(2Z)-2-[(4-Fluoro-3-methylphenyl)imino]-4-[3-methyl-4-(morpholin-4-ylmethyl)phenyl]-1,3-thiazol-3(2H)-yl]ethyl}-N'-methylurea

[Chemical Formula 25]

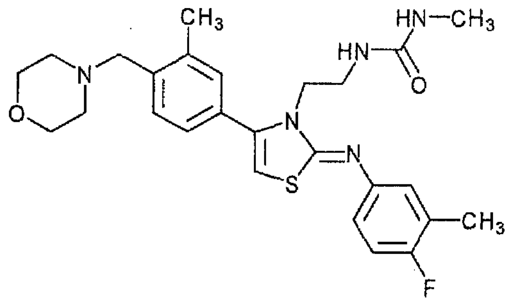

1-[3-Methyl-4-(morpholin-4-ylmethyl)phenyl]ethanone (200 mg) obtained in Reference Example 14 was treated in a similar manner to Example 1 to give the titled compound (57 mg) as a colorless crystal. Melting Point: 139-140°C
$^1$H-NMR($CDCl_3$): δ 2.27(3H, s), 2.41(3H, s), 2.48(4H, m), 2.66(3H, d, J=4.7), 3.42(2H, q, J=6.4, 11.9), 3.49(2H, s), 3.72 (4H, m), 3.87(2H, t, J=6.4), 5.76(1H, s), 6.84-6.91(2H, m), 6.99(1H, t, J=9.0), 7.12(2H, m), 7.37(1H, d, J=7.6)

Reference Example 15

Methyl 2-methoxy-4-methylbenzoate

[Chemical Formula 26]

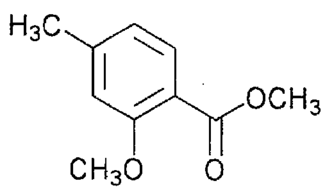

To a solution of 2-hydroxy-4-methylbenzoic acid (4.56 g) in DMF (70 ml) were added potassium carbonate (16.6 g) and methyl iodide (12.8 g), and the mixture was stirred at room temperature overnight. Then, the reaction mixture was poured into ice water (50 ml), and extracted with ethyl acetate. The organic layer was washed with water, then a saturated saline, and then dried over sodium sulfate, and the solvent was evaporated under reduced pressure. The residue was purified by silica gel column chromatography [hexane:ethyl acetate = 2:1] to give the titled compound (5.41 g) as a pale yellow oil.
$^1$H-NMR($CDCl_3$): δ 2.39(3H, s), 3.87(3H, s), 3.90(3H, s), 6.79(2H, m), 7.73(1H, d, J=8.4)

Reference Example 16

Methyl 2-methoxy-4-(morpholin-4-ylmethyl)benzoate

[Chemical Formula 27]

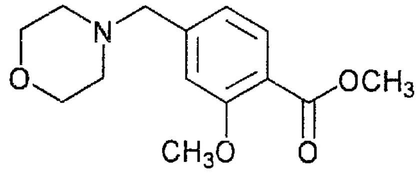

To methyl 2-methoxy-4-methylbenzoate (4.71 g) obtained in Reference Example 15 were added N-bromosuccinimide (5.58 g) and 2,2'-azobis(2-methylpropionitrile) (0.43 g), and the mixture was stirred under nitrogen at 70°C for 7 hours. Thereto was added ether (70 ml). The insoluble was filtered off, and the filtrate was evaporated under reduced pressure. The resulting residue was dissolved in methylene chloride (45 ml), and thereto were added morpholine (2.27 g) and triethylamine (5.28 g) at 0°C. Then, the mixture was stirred at room temperature for 5 hours. Thereto was added water (20 ml), and the mixture was extracted with chloroform. The organic layer was dried over sodium sulfate, and the solvent was evaporated under reduced pressure. The residue was purified by silica gel column chromatography [0.5% methanol-chloroform] to give the titled compound (1.28 g) as a colorless oil. $^1$H-NMR($CDCl_3$): δ 2.45(4H, m), 3.51(2H, s), 3.72 (4H, m), 3.88(3H, s), 3.92(3H, s),6.94(1H, d, J=7.9), 7.00(1H, s), 7.75(1H, d, J=7.9)

Reference Example 17

N,2-Dimethoxy-N-methyl-4-(morpholin-4-ylmethyl)benzamide

[Chemical Formula 28]

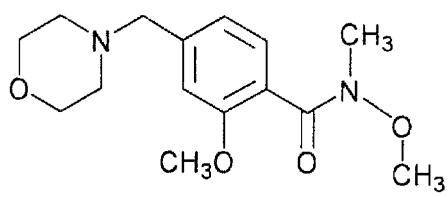

To a solution of methyl 2-methoxy-4-(morpholin-4-ylmethyl)benzoate (1.55 g) obtained in Reference Example 16 in methanol (10 ml) was added 1N aqueous sodium hydroxide solution (10 ml), and the mixture was stirred at room temperature for 5 hours. Thereto was added 1N hydrogen chloride water. The reaction solution was acidified, and then the solvent was evaporated under reduced pressure. To the resulting residue were added N,O-dimethylhydroxylamine hydrochloride (0.63 g), 1-(3-dimethylaminopropyl)-3-ethylcarbodiimide hydrochloride (1.68 g), 1-hydroxybenzotriazole monohydrate (1.34 g) and triethylamine (2.1 ml), and the mixture was stirred at room temperature for 2 hours. The reaction mixture was poured into water (100 ml), and extracted with ethyl acetate. The organic layer was dried over sodium sulfate, and the solvent was evaporated under reduced pressure. The residue was purified by silica gel column chromatography [0.5% methanol-chloroform] to give the titled compound (1.15 g) as a yellow oil.
$^1$H-NMR($CDCl_3$): δ 2.45(4H, m), 3.32-3.55(6H, br), 3.50(2H, s), 3.72(4H, m), 3.86(3H, s), 6.92-6.94(2H, m), 7.21(1H, d, J=7.3)

Reference Example 18

1-[2-Methoxy-4-(morpholin-4-ylmethyl)phenyl]ethanone

[Chemical Formula 29]

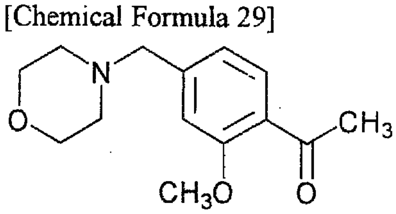

N,2-DimethoxyN-methyl-4-(morpholin-4-ylmethyl)benzamide (1.06 g) obtained in Reference Example 17 was treated in a similar manner to Reference Example 9 to give the titled compound (0.84 g) as a pale yellow oil.
$^1$H-NMR($CDCl_3$): δ 2.45(4H, m), 2.61(3H, s), 3.51(2H, s), 3.73(4H, m), 3.93(3H, s), 6.95(2H, m), 7.70(1H, d, J=7.9)

Example 20

N-{2-[(2Z)-2-[(4-Fluoro-3-methylphenyl)imino]-4-[2-methoxy-4-(morpholin-4-ylmethyl)phenyl]-1,3-thiazol-3(2H)-yl]ethyl}-N'-methylurea

[Chemical Formula 30]

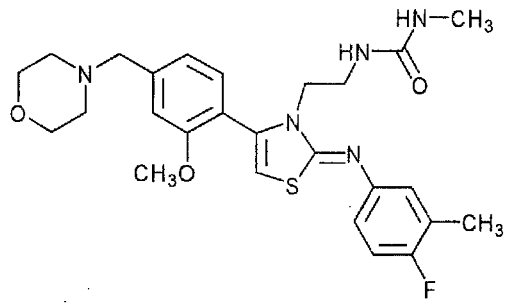

1-[2-Methoxy-4-(morpholin-4-ylmethyl)phenyl]ethanone (125 mg) obtained in Reference Example 18 was treated in a similar manner to Example 1 to give the titled compound (70 mg) as a colorless crystal.
Melting Point: 158-162˚C
$^1$H-NMR($CDCl_3$): δ 2.27(3H, s), 2.49(4H, m), 2.66(3H, d, J=4.7), 3.33(2H, m), 3.54(2H, s), 3.70-3.76(6H, m), 3.86(3H, s), 5.73(1H, s), 6.86-7.00(5H, m), 7.18(1H, t, J=7.51)

Reference Example 19

Methyl 3-methoxy-4-(morpholin-4-ylmethyl)benzoate

[Chemical Formula 31]

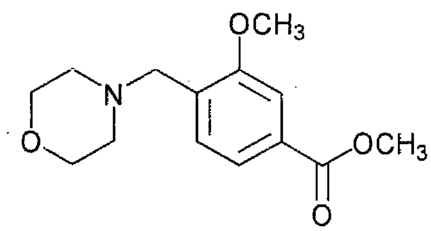

To a solution of methyl 4-(bromomethyl)-3-methoxybenzoate in tetrahydrofuran (35 ml) was added morpholine (5.32 g), and the mixture was stirred at room temperature for 1 day. The solvent was evaporated under reduced pressure, and thereto was added water (5 ml) and the mixture was extracted with ethyl acetate. The organic layer was washed with water, then a saturated saline, and then dried over sodium sulfate. The solvent was evaporated under reduced pressure to give the titled compound (2.65 g) as a colorless oil.
$^1$H-NMR($CDCl_3$): δ 2.50(4H, m), 3.59(2H, s), 3.73(4H, m), 3.88(3H, s), 3.92(3H, s),7.46(1H, d, J=7.8), 7.52(1H, s), 7.63 (1H, d, J=7.8)

Reference Example 20

N,3-Dimethoxy-N-methyl-4-(morpholin-4-ylmethyl)benzamide

[Chemical Formula 32]

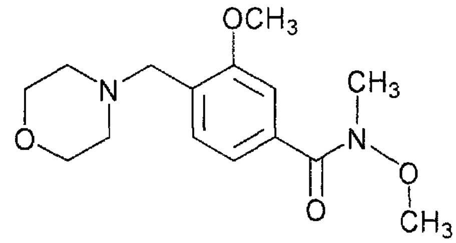

Methyl 3-methoxy-4-(morpholin-4-ylmethyl)benzoate (2.65 g) obtained in Reference Example 19 was treated in a similar manner to Reference Example 17 to give the titled compound (0.88 g) as a pale yellow oil.
$^1$H-NMR($CDCl_3$): δ 2.57(4H, m),3.36(3H, s), 3.59(3H, s),3.64(3H, s), 3.75(4H, m), 3.85(3H, s), 7.20(1H, s), 7.24(1H, d, J=7.8), 7.40(1H, d, J=7.8)

Reference Example 21

1-[3-Methoxy-4-(morpholin-4-ylmethyl)phenyl]ethanone

[Chemical Formula 33]

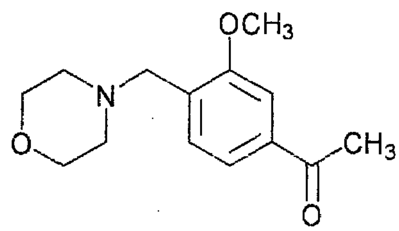

N,3-Dimethoxy-N-methyl-4-(morpholin-4-ylmethyl)benzamide (0.87 g) obtained in Reference Example 20 was treated in a similar manner to Reference Example 9 to give the titled compound (0.54 g) as a pale yellow oil.
$^1$H-NMR($CDCl_3$): δ 2.52(4H, m), 2.61(3H, s), 3.60(2H, s), 3.74(4H, m), 3.89(3H, s), 7.48-7.55(3H, m)

Example 21

N-{2-[(2Z)-2-[(4-Fluoro-3-methylphenyl)imino]-4-[3-methoxy-4-(morpholin-4-ylmethyl)phenyl]-1,3-thiazol-3(2H)-yl] ethyl}-N'-methylurea

[Chemical Formula 34]

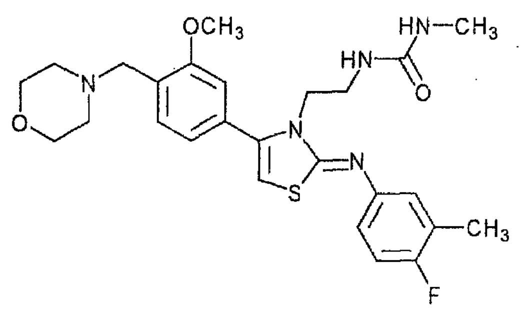

1-[3-Methoxy-4-(morpholin-4-ylmethyl)phenyl]ethanone (125 mg) obtained in Reference Example 21 was treated in a similar manner to Example 1 to give the titled compound (95 mg) as a colorless crystal.
Melting Point: 152-154˚C
$^1$H-NMR($CDC1_3$). δ 2.28(3H, s), 2.54(4H, m), 2.66(3H, d, J=4.7), 3.34(2H, m), 3.58(2H, s), 3.75(4H, m), 3.87-3.90(5H, m), 5.80(1H, s), 6.83-6.94(4H, m), 7.00(1H, t, J=8.9), 7.47(1H, d, J=7.7)

Examples 22 to 33

The acetophenones obtained in Reference Examples 9, 14, 18 and 21 were reacted with various thioureas in a similar manner to Example 1 to give compounds shown in Table 2.

[Table 2]

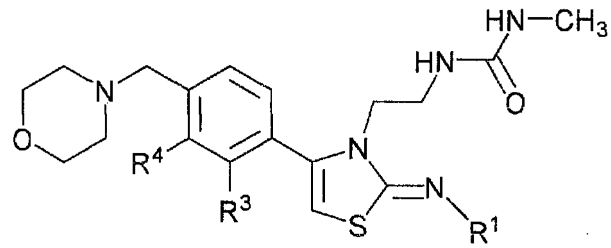

| Example | $R^1$ | $R^3$ | $R^4$ | Yields (%) | Melting Point (˚C) |
|---|---|---|---|---|---|
| 22 | 3-fluoro-4-methylphenyl | Me | H | 44 | 171-174 |
| 23 | 3-fluorophenyl | Me | H | 52 | 119-121 |
| 24 | 4-fluoro-2-methylphenyl | Me | H | 30 | 122-125 |
| 25 | 2,4-difluorophenyl | Me | H | 48 | 154-160 |
| 26 | 2,3-difluorophenyl | Me | H | 37 | 132-135 |
| 27 | 3-fluorophenyl | H | Me | 30 | 151-152 |
| 28 | 3-fluorophenyl | OMe | H | 39 | 163-166 |
| 29 | 3-fluoro-4-methylphenyl | OMe | H | 36 | 144-150 |
| 30 | 2,3-difluorophenyl | OMe | H | 35 | 161-165 |
| 31 | 3-fluorophenyl | H | OMe | 64 | 170-174 |
| 32 | 2,3-difluorophenyl | H | OMe | 63 | 150-153 |
| 33 | 2,4-difluorophenyl | H | OMe | 60 | 164-169 |

Reference Example 22

4-{[(35,5S)-3,5-Dimethylmorpholin-4-yl]methyl}-N-methoxy-N-methylbenzamide

[Chemical Formula 35]

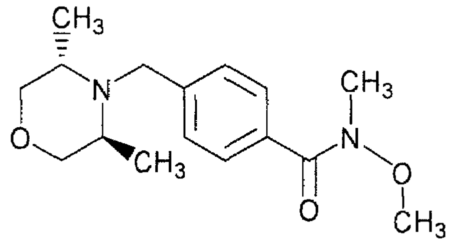

To a solution of 4-carboxybenzoic acid (1.24 g) in DMF (20 ml) were added N,O-dimethylhydroxylamine hydrochloride (0.80 g), 1-(3-dimethylaminopropyl)-3-ethylcarbodiimide hydrochloride (2.38 g), 1-hydroxybenzotriazole monohydrate (1.9 g) and triethylamine (2.9 ml), and the mixture was stirred at room temperature for 2.5 hours. The reaction mixture was poured into water (170 ml), and extracted with ethyl acetate. The organic layer was washed with water, then a saturated saline, and then dried over sodium sulfate. The solvent was evaporated under reduced pressure.
To a solution of the resulting residue in methylene chloride (20 ml) were added (3S,5S)-3,5-dimethylmorpholine hydrochloride (1.05 g) which was synthesized according to the method of Jan Runsink et al., Synthesis, 1994, 1, 66-72 and triethylamine (0.96 ml), then thereto were added sodium triacetoxyborohydride (4.39 g) and acetic acid (0.39 ml), and the mixture was stirred at room temperature overnight. The mixture was neutralized by aqueous saturated sodium bicarbonate solution, and then extracted with chloroform. The organic layer was dried over sodium sulfate, and the solvent was evaporated under reduced pressure. The residue was purified by silica gel column chromatography [0.5% methanol-chloroform to give the titled compound (0.52 g) as a colorless oil.
$^1$H-NMR($CDCl_3$): δ 1.00(6H, d, J=6.5), 2.81(2H, m), 3.36(3H, s), 3.37-3,42(3H, m), 3.57(3H, s), 3.69(2H, dd, J=3.0, 10.8), 3,98(1H, d, J=14.1), 7.41(2H, d, J=8.2), 7.63(2H, d, J=8.2)

Reference Example 23

1-(4-{[(3S,5S)-3,5-Dimethylmorpholin-4-yl]methyl}phenyl)ethanone

[Chemical Formula 36]

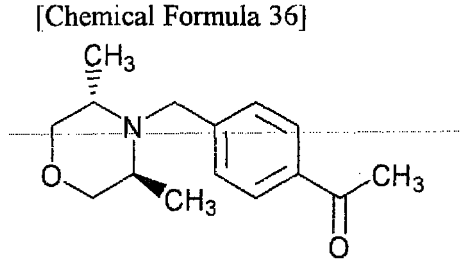

4-{[(3S,5S)-3,5-Dimethylmorpholin-4-yl]methyl}-N-methoxy-N-methylbenzamide (755 mg) obtained in Reference Example 22 was treated in a similar manner to Reference Example 9 to give the titled compound (0.54 g) as a pale yellow oil.
$^1$H-NMR($CDCl_3$): δ 1.00(6H, d, J=6.4), 2.60(3H, s), 2.81(2H, m), 3.37-3,47(3H, m), 3.70(2H, dd, J=3.0, 10.9), 3.99(1H, d, J=14.4), 7.48(2H, d, J=8.2), 7.90(2H, d, J=8.2)

Example 34

N-{2-[(2Z)-4-(4-{[(3S,5S)-3,5-Dimethylmorpholin-4-yl]methyl}phenyl)-2-[(4-fluoro-3-methylphenyl)imino]-1,3-thiazol-3(2H)-yl]ethyl}-N'-methylurea

[Chemical Formula 37]

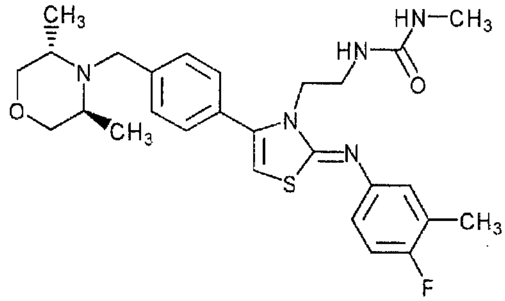

1-(4-1[(3S,5S)-3,5-Dimethylmorpholin-4-yl]methyl}phenyl)ethanone (132 mg) obtained in Reference Example 23 was treated in a similar manner to Example I to give the titled compound (63 mg) as a colorless crystal.
Melting Point: 11.6-119˚C
$^1$H-NMR($CDCl_3$): δ 1.03(6H, d, J=6.4), 2.28(3H, s), 2.66(3H, d, J=4.7), 2.85(2H, m), 3.39-3.46(5H, m), 3.71(2H, dd, J=3.0, 10.9), 3.86(2H, t, J=6.5), 4.00(1H, d, J=14.3), 5.77(1H, s), 6.84-6.92(2H, m), 6.99(1H, t, J=8.9), 7.27(2H, d, J=8.0), 7.48(2H, d, J=8.0)

Reference Example 24

4-{[(3R,5S)-3,5-Dimethylmorphlin-4-yl]methyl}-N-methoxy-N-methylbenzamide

[Chemical Formula 38]

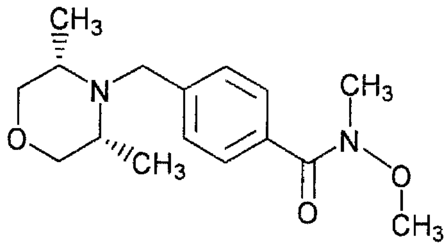

(3R,5S)-3,5-Dimethylmorpholine hydrochloride (1.21 g) was treated in a similar manner to Reference Example 22 to give the titled compound (0.62 g) as a colorless oil.
$^1$H-NMR($CDCl_3$): δ 0.90(6H, d, J=6.2), 2.65(2H, m), 3.26(2H, t, J=10.7), 3.36 (3H, s), 3.57(3H, s), 3.70(2H, dd, J=2.8, 11.4), 3,81(2H, s), 7.41(2H, d, J=8.3), 7.63(2H, d, J=8.3)

Reference Example 25

1-(4-{[(3R,5S)-3,5-Dimethylmorpholin-4-yl]methyl}phenyl)ethanone

[Chemical Formula 39]

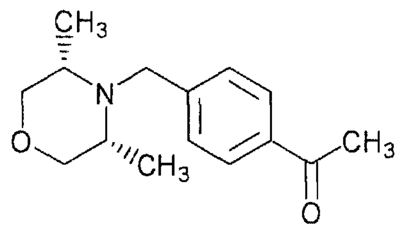

4-{[(3R,5S)-3,5-Dimethylmorpholin-4-yl]methyl}-N-methoxy-N-methylbenzamide (620 mg) obtained in Reference Example 34 was treated in a similar manner to Reference Example 9 to give the titled compound (0.42 g) as a pale yellow oil. $^1$H-NMR($CDCl_3$): δ 0.89(6H, d, J=6.2), 2.60(3H, s), 2.63(2H, m), 3.27(2H, t, J=10.3), 3.71(2H, dd, J=2.9, 11.5), 3,82(2H, s), 7.49(2H, d, J=8.4), 7.90(2H. d, J=8.4)

Examples 35 to 40

The acetophenones obtained in Reference Examples 23 and 25 were reacted with various thioureas in a similar manner to Example 1 to give compounds shown in Table 3.

[Table 3]

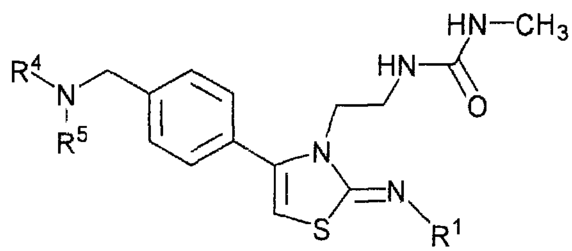

| Example | $R^1$ | $R_4(R_5)N$ | Yields (%) | Melting Point (˚C) |
|---|---|---|---|---|
| 35 | 3-fluoro-4-methylphenyl | (3S, 5S)-3,5-dimethylmorpholino | 47 | 137-140 |
| 36 | 3-fluorophenyl | (3S, 5S)-3,5-dimethylmorpholino | 60 | 128-130 |
| 37 | 2,3-difluorophenyl | (3S, 5S)-3,5-dimethylmorpholino | 57 | 131-133 |
| 38 | 4-fluoro-43-methylphenyl | (3S, 5R)-3,5-dimethylmorpholino | 65 | 139-142 |
| 39 | 3-fluoro-4-methylphenyl | (3S, 5R)-3,5-dimethylmorpholino | 58 | 155-157 |
| 40 | 2,3-difluorophenyl | (3S, 5R)-3,5-dimethylmorpholino | 69 | 156-160 |

Reference Example 26

Methyl 3-(morpholin-4-ylmethyl)benzoate

[Chemical Formula 40]

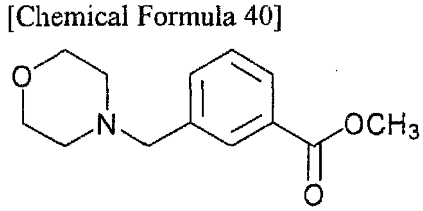

To a solution of methyl 3-(bromomethyl)benzoate (3.03 g) in tetrahydrofuran (40 ml) was added morpholine (3.45 ml), and the mixture was stirred at room temperature for 4 hours. The solvent was evaporated under reduced pressure, and to the residue was added water (5 ml) and the mixture was extracted with ethyl acetate. The organic layer was washed with water, then a saturated saline, and then dried over sodium sulfate. The residue was evaporated under reduced pressure to give the titled compound (3.11 g) as a colorless oil.
$^1$H-NMR($CDCl_3$): δ 2.45(4H. m), 3.54(2H, s), 3.71(4H, m), 3.92(3H, s), 7.40(1H, t, J=7.7), 7.55(1H d, J=7.7), 7.94(1H, td, J=1.4, 7.7), 7.99(1H, s)

Reference Example 27

N-Methoxy-N-methyl-3-(morpholin-4-ylmethyl)benzamide

[Chemical Formula 41]

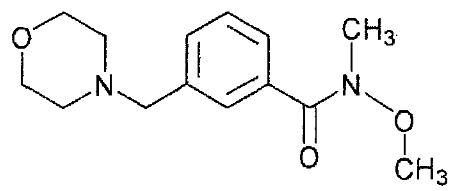

Methyl 3-(morpholin-4-ylmethyl)benzoate (3.11 g) obtained in Reference Example 26 was treated in a similar manner to Reference Example 17 to give the titled compound (3.50 g) as a pale yellow oil.
$^1$H-NMR($CDCl_3$): δ 2.45(4H, m),3.36(3H, s), 3.53(2H, s), 3.56(3H, s), 3.71(4H, m), 7.35(1H, t, J=7.6), 7.45(1H d, J=7.6), 7.94(1H d, J=7.6), 7.63(1H, s)

Reference Example 28

1-[3-(Morpholin-4-ylmethyl)phenyl]ethanone

[Chemical Formula 42]

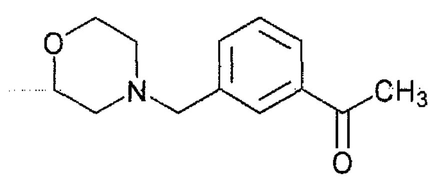

N-Methoxy-N-methyl-3-(morpholin-4-ylmethyl)benzamide (3.50 g) obtained in Reference Example 27 was treated in a similar manner to Reference Example 9 to give the titled compound (1.98 g) as a pale yellow oil.
$^1$H-NMR($CDCl_3$): δ 2.45(4H, m), 2.62(3H, s), 3.55(2H, s), 3.72(4H, m), 7.43(1H, t, J=7.7), 7.56(1H. d, J=7.7), 7.85(1H, td, J=1.4, 7.7), 7.92(1H, s)

Example 41

N-{2-[(2Z)-2-[(4-Fluoro-3-methylphenyl)imino]-4-[3-(morpholin-4-ylmet.hyl)phenyl]-1,3-thiazol-3(2H)-yl]ethyl}-N'-methylurea

[Chemical Formula 43]

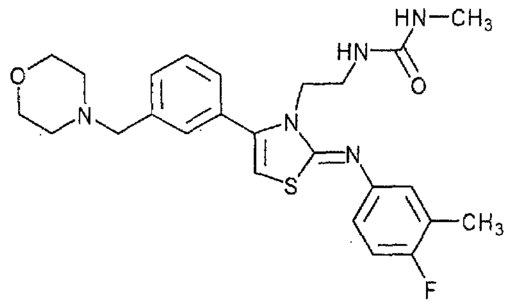

1-[3-(Morpholin-4-ylmethyl)phenyl]ethanone (110 mg) obtained in Reference Example 28 was treated in a similar manner to Example 1 to give the titled compound (40 mg) as a pale yellow oil. $^1$H-NMR($CDCl_3$): δ 2.28(3H, s), 2.47(4H, m), 2.67 (3H, d, J=4.7), 3.44(2H, q, J=6.4,12.8), 3.55(2H, s), 3.73(4H, m), 3,86(2H. t, J=6.4), 5.80(1H, s), 6.84-6.92(2H, m), 6.99 (1H, t J=9.0), 7.24-7.45(4H, m)

Examples 42 to 43

The acetophenone obtained in Reference Example 28 was reacted with various thioureas in a similar manner to Example 1 to give compounds shown in Table 4.

[Table 4]

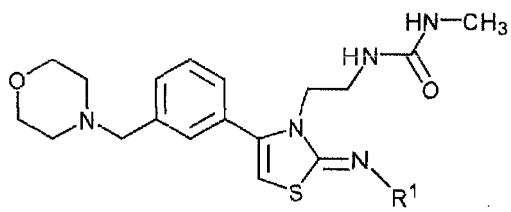

| Example | $R^1$ | Yields (%) | Melting Point (˚C) |
|---|---|---|---|
| 42 | 3-fluorophenyl | 33 | oil |
| 43 | 2,4-difluorophenyl | 29 | oil |

Reference Example 29

4-{2-(4-(Morpholin-4-y-ylcarbonyl)phenyl]ethyl}morpholine

[Chemical Formula 44]

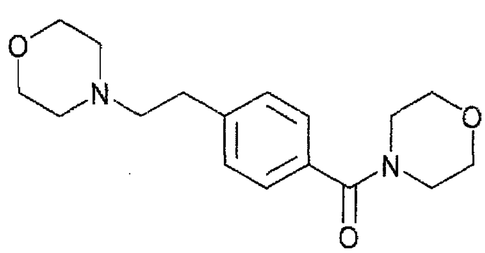

To a solution of 4-(2-bromoethyl)benzoic acid (2.29 g) in methylene chloride (12 ml) were added thionyl chloride (2.38 g) and N,N-dimethylformamide (0.1 ml), and the mixture was heated to reflux for 30 minutes. The reaction mixture was evaporated under reduced pressure, azeotroped with toluene (20 ml) twice, and then dissolved in tetrahydrofuran (50

ml). Then, thereto was added morpholine (1.74 ml) at 0˚C, and then the mixture was stirred at room temperature for 15 minutes. To the reaction mixture was added aqueous saturated sodium bicarbonate solution (10 ml), and then the mixture was extracted with ethyl acetate. The organic layer was washed with water, then a saturated saline, and then dried over sodium sulfate. The solvent was evaporated under reduced pressure. To the resulting residue was added morpholine (10 ml), and the mixture was stirred at 40˚C for 2 hours. The reaction solution was poured into water, and the mixture was extracted with ethyl acetate. The organic layer was dried over sodium sulfate, and the solvent was evaporated under reduced pressure. The residue was purified by silica gel column chromatography [3% methanol-chloroform] to give the titled compound (2.13 g) as a colorless crystal.
$^{1}$H-NMR($CDCl_3$): δ 2.53(4H, m), 2.61(2H, m), 2.83(2H, m), 3.48-3.79(12H, m), 7.25(2H, d, J=8.1), 7.34(2H, d, J=8.1)

Reference Example 30

1-[4-(2-Morpholin-4-ylmethyl)phenyl]ethanone

[Chemical Formula 45]

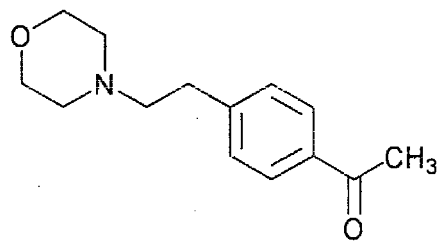

4-{2-[4-(Morpholin-4-ylcarbonyl)phenyl]ethyl}morpholine (2.13 g) obtained in Reference Example 29 was treated in a similar manner to Reference Example 9 to give the titled compound (0.47 g) as a colorless crystal.
$^{1}$H-NMR($CDCl_3$): δ 2.53(4H, m), 2.59(3H, s), 2.63(2H, m), 2.86(2H, m), 3.74(4H, m), 7.30(2H, d, J=8.2), 7.90(2H, d, J=8.2)

Example 44

N-{2-[(2Z)-2-[(4-Fluoro-3-methylphenyl)iminol-4-|4-(2-morpholin-4-ylethyl)phenyl]-1,3-thiazol-3(2H)-yl]ethyl}-N'-methylurea

[Chemical Formula 46]

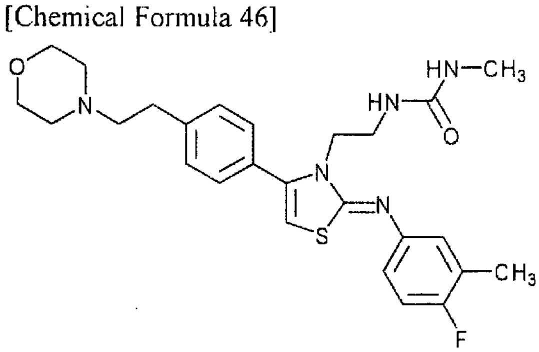

1-[4-(2-Morpholin-4-ylmethyl)phenyl]ethanone (117 mg) obtained in Reference Example 30 was treated in a similar manner to Example 1 to give the titled compound (65 mg) as a colorless crystal. Melting Paint: 167-171˚C
$^{1}$H-NMR($CDCl_3$): δ 2.27(3H, s), 2.55(4H, m), 2.61-2.67(5H, m), 2.86(2H, m), 3.43(2H, q, J=6.4, 11.8), 3.77(4H, m), 3,86 (2H, t, J=6.5), 5.80(1H, s), 6.84-6.92(2H, m), 6.99(1H, t J=9.0), 7.24-7.32(4H, m)

Examples 45 to 47

The acetophenone obtained in Reference Example 30 was reacted with various thioureas in a similar manner to Example 1 to give compounds shown in Table 5.

[Table 5]

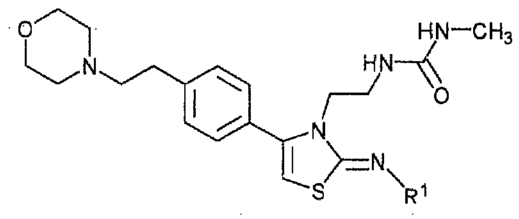

| Example | $R^1$ | Yields (%) | Melting Point (˚C) |
|---|---|---|---|
| 45 | 3-fluorophenyl | 63 | 154-160 |
| 46 | 2, 3-difluorophenyl | 58 | 155-164 |
| 47 | 2, 4-difluorophenyl | 58 | 166-172 |

Reference Example 31

4-Acetyl-N-methoxy,-N-methylbenzamide

[Chemical Formula 47]

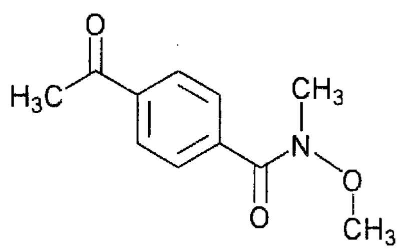

To a solution of 4-acetylbenzoic acid (1.64 g) in DMF (35 ml) were added N,O-dimethylhydroxylamine hydrochloride (1.27 g), 1-(3-dimethylaminopropyl)-3-cthylcarbodiimide hydrochloride (2.88 g), 1-hydroxybenzotriazole monohydrate (2.30 g) and triethylamine (3.5 ml), and the mixture was stirred at room temperature overnight. The reaction mixture was poured into water (150 ml), and the mixture was extracted with ethyl acetate. The organic layer was washed with water, then a saturated saline, and then dried over sodium sulfate. The solvent was evaporated under reduced pressure. The residue was purified by silica gel column chromatography [chloroform] to give the titled compound (2.07 g) as a colorless oil.
1H-NMR($CDCl_3$): δ 2.64(3H, s),3.38(3H, s), 3.54(3H, s), 7.75(2H, dd, J=1.7, 6.7), 8.00(2H, dd, J=1.7, 6.7)

Reference Example 32

N-Methoxy-N-methyl-4-(1-morpholin-4-ylmethyl)benzmide

[Chemical Formula 48]

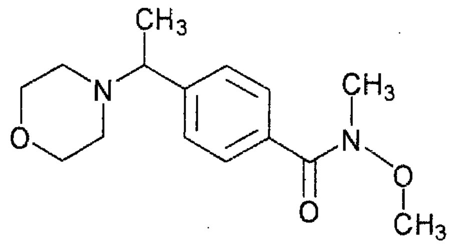

To a solution of 4-acetyl-N-methoxy-N-methylbenzamide (2.07 g) obtained in Reference Example 31 in 1,2-dichloroethane (50 ml) were added morpholine (0.96 g), sodium triacetoxyborohydride (3.18 g) and acetic acid (0.57 ml), and the mixture was stirred at room temperature for 4 days. The mixture was neutralized by aqueous saturated sodium bicarbonate solution, and then extracted with chloroform. The organic layer was dried over sodium sulfate, and the solvent was evaporated under reduced pressure. The residue was purified by silica gel column chromatography [1% methanol-chloroform] to give the titled compound (1.60 g) as a pale yellow oil.
$^1$H-NMR($CDCl_3$): δ 1.34(3H, d, J=6.7), 2.35(2H, m), 2.49(2H, m), 3.33(1H, q, J=6.7)3.36(3H, s), 3.58(3H, s), 3.69(4H, m), 7.36(2H, d, J=8.2), 7.64(2H, d, J=8.2)

Reference Example 33

1-[4-(1-Morpholin-4-ylethyl)phenyl]ethanone

[Chemical Formula 49]

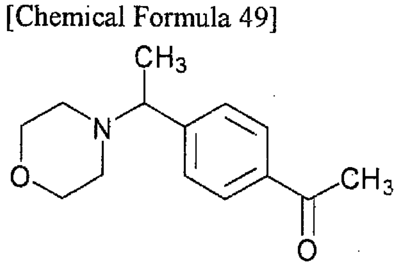

N-Methoxy-N-methyl-4-(1-morpholin-4-ylmethyl)benzamide (1.53 g) obtained in Reference Example 32 was treated in a similar manner to Reference Example 9 to give the titled compound (0.72 g) as a pale yellow oil.
$^1$H-NMR($CDCl_3$): δ 1.34(3H, d, J=6.6), 2.35(2H, m), 2.49(2H, m), 2.60(3H, s), 3.36(1H, q, J=6.6), 3.69(4H, m), 7.43(2H, d, J=8.3), 7.92(2H, d, J=8.3)

Example 48

N-{2-[(2Z)-2-[(4-Fluoro-3-met hylphenyl)imino]-4-[4-(1-motpholin-4-ylethyl)phenyl]-1,3-thiazol-3(2H)-yl]ethyl}-N'-methylurea

[Chemical Formula 50]

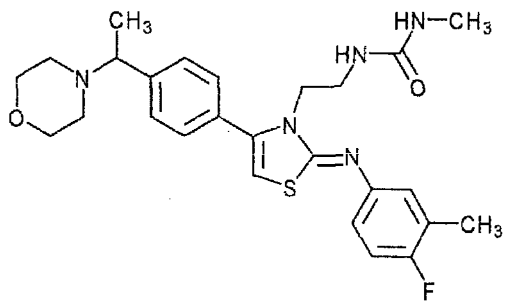

1-[4-(1-Morpholin-4-ylethyl)phenyl]ethanone (140 mg) obtained in Reference Example 33 was treated in a similar manner to Example 1 to give the titled compound (130 mg) as a pale yellow oil. $^1$H-NMR($CDCl_3$): δ 1.37(3H, d, J=6.7), 2.28(3H, s), 2.40(2H, m), 2.51(2H, m), 2.66(3H, d, J=4.7), 3.37(1H, q, J=6.7), 3.44(2H, m), 3.72(4H, m), 3.88(2H, t, J=6.7), 5.78 (1H, s), 6.84-6.92(2H, m), 6.99(1.H, t J=8.8), 7.30(2H, d, J=8.2), 7.42(2H, d, J=8.2)

Examples 49 to 51

The acetophenone obtained in Reference Example 33 was reacted with various thioureas in a similar manner to Example I to give compounds shown in Table 6.

[Table 6]

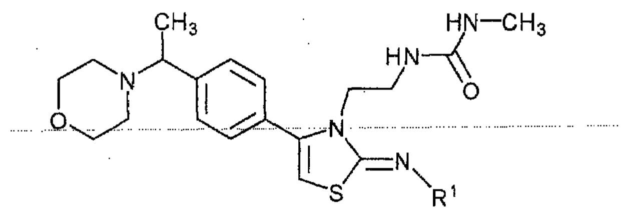

| Example | $R^1$ | Yields (%) | Melting Point (˚C) |
|---|---|---|---|
| 49 | 3-fluorophenyl | 52 | oil |
| 50 | 2,3-difluorophenyl | 72 | oil |
| 51 | 2,4-difluorophcnyl | 72 | oil |

Reference Example 34

1-[4-(Morpholin-4-ylcarbonyl)phenyl]ethanone

[Chemical Formula 51]

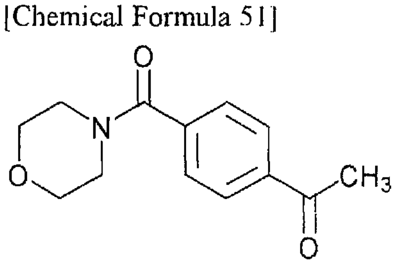

To a solution of 4-acetylbenzoic acid (1.64 g) in DMF (30 ml) were added morpholine (0.87 g), 1-(3-dimethylaminopropyl)-3-ethylcarbodiimide hydrochloride (2.88 g), 1-hydroxybenzotriazole monohydrate (2.30 g) and triethylamine (2.8 ml), and the mixture was stirred at room temperature overnight. The reaction mixture was poured into water (300 ml), and the mixture was extracted with ethyl acetate. The organic layer was washed with water, then a saturated saline, and then dried over sodium sulfate. The solvent was evaporated under reduced pressure. The residue was purified by silica gel column chromatography [chloroform] to give the titled compound (1.52 g) as a colorless crystal.
$^1$H-NMR($CDCl_3$): δ 2.63(3H, s), 3.40-3.80(8H, br), 7.50(2H, td, J=1.6, 1.9,8.4), 8.01(2H, td, J=1.6, 1.9, 8.4)

Example 52

N-{2-[(2Z)-2-[(3-Fluorophenyl)imino]-4-[4-(morpholin-4-ylcarbonyl)phenyl]-1,3-thiazol-3(2H)-yl]ethyl} -N'-methylurea

[Chemical Formula 52]

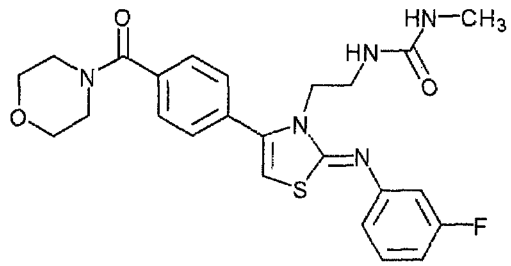

1-[4-(Morpholin-4-ylcarbonyl)phenyl]ethanone (125 mg) obtained in Reference Example 34 and N-[2-({[3-fluorophenyl) amino]carbonothioyl}amino)ethyl]-N'-methylurea (108 mg) were treated in a similar manner to Example 1 to give the titled compound (118 mg) as a colorless crystal. $^1$H-NMR($CDCl_3$): δ 2.67(3H, d, J=4.8), 3.38-3.95(1.2H, m), 5.86(1H, s), 6.79-6,83(2H, m), 6.88(1H, dd, J=0.8, 8.0), 7.31(1H, q, J=6.8), 7.45(2H, d, J=6.4), 7.52(2H, d, J=6.4)

Examples 53 to 59

The acetophenone obtained in Reference Example 34 was reacted with various thioureas in a similar manner to Example I to give compound shown in Table 7.
[Table 7]

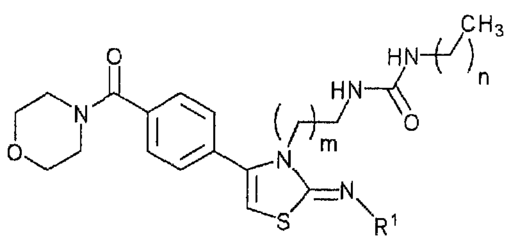

| Example | $R^1$ m | | n | Yields(%) | Melting Point (˚C) |
|---|---|---|---|---|---|
| 53 | 4-fluoro-3-methylphenyl | 1 | 0 | 45 | 183-186 |
| 54 | 2,4-difluorophenyl | 1 | 0 | 53 | 179-180 |
| 55 | 3-fluoro-4-methylphenyl | 1 | 0 | 58 | 175-178 |
| 56 | 3-chlorophenyl | 1 | 0 | 42 | 182-186 |
| 57 | 3-fluorophenyl | 2 0 | | 45 | 172-177 |
| 58 | 4-fluoro-3-methylphenyl | 2 0 | | 54 | 196-203 |

(continued)

| Example | $R^1$ m | | n | Yields(%) | Melting Point (˚C) |
|---|---|---|---|---|---|
| 59 | 3-chlorophenyl | 2 | 1 | 36 | 191-198 |

Reference Examples 35 to 36

4-Acetylbenzoic acid was reacted with various amines in a similar manner to Reference Example 34 to give compounds shown in Table 8.

[Table 8]

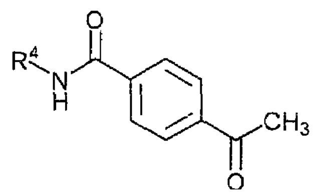

| Reference Example | $R^4$ | Yields (%) | Melting Point (˚C) |
|---|---|---|---|
| 35 | ethyl | 68 | oil |
| 36 | cyclopropyl | 77 | oil |

Examples 60 to 64

The acetophenones obtained in Reference Examples 35 and 36 were reacted with various thioureas in a similar manner to Example 1 to give compounds shown in Table 9.

[Table 9]

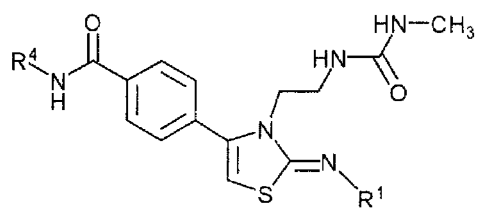

| Example | $R^1$ | $R^4$ | Yields (%) | Melting Point (˚C) |
|---|---|---|---|---|
| 60 | 3-fluorophenyl | ethyl | 55 | 170-176 |
| 61 | 4-fluorophenyl | ethyl | 48 | 172-178 |
| 62 | 3,4-difluorophenyl | ethyl | 69 | 180-188 |
| 63 | 3-chlorophenyl | ethyl | 58 | 185-192 |
| 64 | 3-fluorophenyl | cyclopropyl | 63 | 179-185 |

Pharmacological Tests

Test 1

Receptor-Binding Assessment Test of Compounds Using Rat Lung Membranes

The assessment test was carried out according to the method of Sugasawa, T. et al., J. Biol. Chem., 272, 21244-21252 (1997).

Preparation of Rat Lung Membranes

Lung taken out from SD male rat (7-week old under test, Charles River Laboratories Japan, Inc.) was removed trachea and blood vessels to shred and washed with iced tris-saline buffer (10 mM tris hydrochloric acid-154 mM sodium chloride, pH7.4). The resultant was homogenized by Hiscotron with ice-cold tris-saline buffer containing a homogenization buffer (1 mM ethylenediaminetetraacetic acid (EDTA), 1 mM 4-(2-aminoethyl)benzenesulfonyl fluoride (AEBSF), 5 μg/ml aprotinin, 5 μg/ml leupeptine) (maximum rate: 1 minute). The supernatant after low-speed centrifugation (1500 x g, 20 minutes, 4˚C) was ultracentrifuged (100000 x g, 20 minutes, 4˚C), and the pellet was suspended in tris-saline buffer to store at -80˚C. The protein concentrations were determined by Bio-Rad Protein Assay Kit using bovine serum albumin (BSA) as a standard.

Ligand Binding Assay

To each well in protein-nonabsorptive round-bottom 96-well assay plate (commercially available from Iwaki Glass Co., Ltd.) were added tris-saline buffer (200 μl) containing 1 nM [$^{125}$I]-iodocyanopindolol (commercially available from Amersham plc), 10 μM serotonine, 20 μm dl-propranolol, 10 μM phentolamine, 1.1 mM ascorbic acid and 100 μg lung membrane, and the mixture was pipetted to combine and then incubated at 37˚C for 30 minutes. The test compounds were dissolved in 100% dimethyl sulfoxide solution, and thereto was added 2 μl (final DMSO concentration: 1%). In order to calculate nonspecific binding amounts, to the mixture was added L-threo-3-(3,4-dihydroxyphenyl)-N-[3-(4-fluorophenyl)propyl]serine pyrrolidine amide at 100 μM of the final concentration as an alternative to the test compounds. During this time, 100 μl of 0.3% polyethyleneimine (PEI)/tris-saline buffer was added to MultiScreen plate (96-well B glass fiber, Millipore Cat. No. MAFB NOB10), and incubated for over 30 minutes. The resultant was filtered with aspiration (aspiration by the addition of 200 μl iced tris-saline buffer) to wash, and the reaction solutions on 96-well assay plate were filtered with aspiration to wash four times on MultiScreen plate B glass fiber filter paper at the bottom of MultiScreen plate was punched out, and γ-ray doses of [$^{125}$I]-iodocyanopindolol trapped on the filter paper were determined as binding amounts. The binding amounts in the presence of DMSO (final concentration: 1%) were total binding amounts, and the binding amounts in the presence of 100 μM L-threo-3-(3,4-dihydroxyphenyl)-N-[3-(4-fluorophenyl)propyl]serine pyrrolidine amide were nonspecific binding amounts. The values subtracted the nonspecific binding amounts from the total binding amounts were the specific binding amounts. The binding activity of a compound was calculated according to the following formula, which was shown in the ratio of inhibiting the specific binding of [$^{125}$I]-iodocyanopindolol to rat lung membrane SMBS by the test compound.

[Formula 1]

Binding Activity of Test Compound (%)

$$= \left\{ 1 - \frac{(\text{Binding Amounts in the Presence of Test Compound}) - (\text{Nonspecific Binding Amounts})}{(\text{Total Binding Amounts}) - (\text{Nonspecific Binding Amounts})} \right\} \times 100$$

<u>Test 2</u>

Assessment Test of Steroid Hormone Synthesis Inhibition

(1) Materials

Cell Strainer (BD Falcon®)
Cell Titer-Glo® (Promega)
Collagenase from Clostridium histolyticum (Sigma-Aldrich)
Corticosterone EIA kit (Cayman)
Dimethylsulfoxide (referred to as DMSO hereinafter) (nacalai tesque)
D-MEM (GIBCO)

DNase I (Invitrogen)
Fetal Bovine Serums (referred to as FBS hereinafter) (Bioflud)
Multiple well plates for adhesive cell culture, 96 wells (IWAKI)
$N^6$,2'-0-Dibutyryladenosine 3',5'-cyclic monophosphatc sodium salt (referred to as cAMP hereinafter) (Sigma)
Penicillin Streptomycin Mixed Solution (nacalai tesque)

Control examples 1 to 4: Control example I was Example 350 of Patent Document 2. Control examples 2 to 4 were synthesized according to Patent Document 2.

(2) Methods

Adrenal glands were taken out from rats, and removed excess connective tissues, adiposes and membranes in D-MEM. The adrenal glands were transferred to polytubes, and quickly segmentalized with scissors. Thereto was added D-MEM containing 1 mg/mL collagenase and 0.25 μL DNase I, and then the mixture was shaken at 37°C for 10 minutes in 80 times/minute. Then, the mixture was pipetted by 1 mL truncated pipette tip, and shaken again under the same condition. After 10 minutes, the mixture was pipetted again by 1 mL pipette tip, and the cell suspension was filtered through metal mesh. The filtrate was centrifuged at 4°C for 5 minutes in 500 g. The supernatant was removed, and thereto was added a mixed solution of 0.16 M $NH_4Cl$ and 0.17 M Tris-HCl in 3 mL. The mixture was let stand on ice for 5 minutes, and then centrifuged under the same condition. Then, the supernatant was removed and then thereto was added D-MEM. The cell suspension was prepared and centrifuged under the same condition. Again, the supernatant was removed and then thereto was added D-MEM to give a cell suspension. The cell suspension was filtered by Cell Strainer (BD Falcon®). The filtrate was centrifuged under the same condition. After the completion of the centrifugation, the supernatant was removed, and thereto was added D-MEM containing 1% Penicillin Streptomycin Mixed Solution and 10% FBS. The mixture was seeded in Multiple well plate for adhesive cell culture, 96 wells (IWAKI) in 10000 cells/well, and incubated at 37°C overnight in $CO_2$ incubator.

Test article concentrations were prepared in DMSO to 2000 μM, and D-MEM containing 20 μM test article in D-MEM was prepared.
Cell-seeded plate was incubated overnight, and then the medium was removed and thereto was added D-MEM containing 20 μM test article in 50 μL/well. Then, to the mixture was promptly added D-MEM containing 20 μm cAMP in 50 μL/well so that the final concentration of the test article was 10 μM. After the addition of 2 types of D-MEM, the mixture was incubated at 37°C for 2 hours in $CO_2$ incubator. After 2 hours, the supernatant (50 μL) of the medium was collected. For cells, the cell toxicity of the test article was assessed according to a conventional method using Cell Titcr-Glo® (Promega). For the collected supernatant, the Corticosterone concentration in the supernatant was determined according to a conventional method using Corticosterone EIA kit (Cayman).
The inhibition rate of 10 μM test article to Corticosterone generation was calculated according to the following formula confirming that the cell toxicity by the test article was not recognized.

[Formula 2]

$$\text{Inhibition Rate (\%)} = 100 - \left\{ \frac{\text{(Corticosterone Concentration of Supernatant Exposed to Test Article)}}{\text{(Corticosterone Concentration of Supernatant Exposed to DMSO Only)}} \right\} \times 100$$

The results of the above Tests 1 and 2 are shown in Table 10.
Binding Activities (Effects) and Steroid Synthesis Inhibition Rates (By-Effects) of Example Compounds

[Table 10]

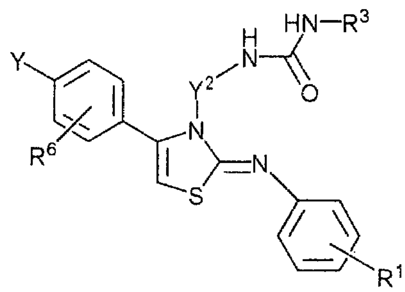

| Example | $R^1$ | Y | $R^6$ | $Y^2$ | $R^3$ | Binding Activity (%) (1 μM) | Steroid Synthesis Inhibition Rate (%) (10 μM) |
|---|---|---|---|---|---|---|---|
| Control Example 1 | 4-fluoro | morpholino | H | ethylene | methyl | 88 | 57 |
| Control Example 2 | 4-trifluoro-methyl | morpholino | H | ethylene | methyl | 81 | 73 |
| Control Example 3 | diisopropyl | morpholino | H | ethylene | methyl | 83 | 52 |
| Control Example 4 | 3-fluoro | morpholino | 2.6-difluoro | ethylene | methyl | 92 | 95 |
| 18 | 4 methylmethyl | 4-morpholinomethyl | 2-methyl | ethylene | methyl | 88 | 28 |
| 21 | 4-fluoro-3-methyl | 4-morpholinomethyl | 3-methoxy | ethylene | methyl | 82 | 8 |
| 52 | 3-fluoro | 4-morpholinocarbonyl | H | ethylene | methyl | 59 | 0 |
| 59 | 3-chloro | 4-morpholinocarbonyl | H | ethylene | ethyl | 66 | 7 |
| 61 | 4-fluoro | 4-ethylaminocarbonyl | H | ethylene | methyl | 62 | 6 |
| 62 | 3,4-difluoro | 4-ethylamillcarbonyl ethylaminocarbonyl | H | ethylene | methyl | 65 | 2 |

Test 3

Assessment of Compounds in Guinea Pig Late Asthma Model

The assessment was carried out using a compound obtained in Example 52.

Hartley male guinea pigs (commercially available from Japan SLC, Inc.) were pre-bred about 1 week after arrival, and then exposed by inhalation to 2% (w/v) ovalbumin (OA) saline in a plastic box (4 guniea pigs/box) for 5 minutes using a ultrasonic nebulizer (OMRON NE-U12, condition: maximum nebulizing amounts, maximum air volume), and sensitized (day 0). The same operation was carried out on day 7. On day 14 or 15, each guinea pig was administered 2% OA by inhalation for 5 minutes to initiate a response (challenge). One hour before the challenge, an antihistamine agent pyrilamine maleate (dissolved in saline, 10 mg/2 ml/kg) was intraperitoneally administered. A test compound was suspended in 0.5% methylcellulose (MC), and was orally administered one hour before the antigen challenge in 3 mg/5 ml/kg 0.5% MC was administered to a control group in a similar manner.
A determination and analysis of respiratory function were carried out according to the method of Penny A. Hutson et al.

Am Rev Respir Dis 1988 137, 548-557. The respiratory function was determined before the antigen challenge (before drug administration) and 5 minutes and 1, 2, 3, 4, 5, 6, 7 and 8 hours after the antigen challenge, and wave profiles were imported using MacLab Chart v3.4 (AD Instruments) and analyzed later. The assessment was carried out on the basis of sRaw/TV by subtracting the former sRaw/TV value from sRaw/TV value for each animal at each point of time. The sRaw/TV values subtracting the former values were plotted against measured times, and each area under the curve ($AUC_{4-8hr}$) of each animal was calculated to compare each other in the range of 4 to 8 hours after the antigen challenge. The statistical analysis was carried out using SAS preclinical package Version 5.0. It was evaluated by Dunnett-type multiple comparison whether or not there were any significant differences between a control group and each drug administration group.
The results are shown in Table 11.

[Table 11]

| | $AUC_{4-8hr}$ ((sRaw/TV)•h) | Improvement Rate (%) |
|---|---|---|
| Control Group | 0.925 | 0 |
| Example 52 Compound | 0.427 | 54 |

Example 52 compound had an improvement rate of 54%, and a significant difference was shown compared to the control group ($p<0.01$; Dunnett's test).

INDUSTRIAL APPLICABILITY

The 5-membercd ring compound or a pharmaceutically acceptable salt thereof of the present invention inhibits infiltration of leukocytes including eosinophils, lymphocytes, etc., and hence, is useful for the treatment of various inflammations.

## Claims

1. A 5-membered ring compound of formula (1):

[Chemical Formula 1]

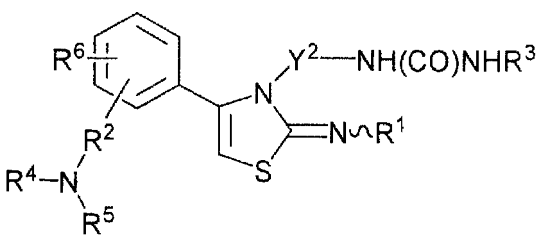

wherein $R^1$ is phenyl optionally substituted by a halogen atom, $C_1$-$C_3$ alkyl or $C_1$-$C_3$ alkoxy;
$R^2$ is $C_1$-$C_3$ alkylene optionally substituted by $C_1$-$C_3$ alkyl, or carbonyl;
$R^3$ is $C_1$-$C_3$ alkyl;
$R^4$ and $R^5$ are each independently hydrogen atom or $C_1$-$C_3$ alkyl; or $-N(R^4)R^5$ may be morpholino optionally substituted by $C_1$-$C_3$ alkyl;
$Y^2$ is $C_2$-$C_4$ alkylene;
$R^6$ is hydrogen atom, a halogen atom, $C_1$-$C_3$ alkyl or $C_1$-$C_3$ alkoxy; or a pharmaceutically acceptable salt thereof.

2. The 5-membered ring compound of claim 1, wherein $-N(R^4)R^5$ is morpholino optionally substituted by $C_1$-$C_3$ alkyl, or a pharmaceutically acceptable salt thereof.

3. The 5-membered ring compound of claim 1, wherein $-N(R^4)R^5$ is morpholino, or a pharmaceutically acceptable salt thereof.

**4.** The 5-membered ring compound of claim 1, wherein $R^2$ is methylene, or a pharmaceutically acceptable salt thereof.

**5.** The 5-membered ring compound of claim 1, wherein $R^1$ is phenyl optionally substituted by a halogen atom, or a pharmaceutically acceptable salt thereof.

**6.** The 5-membered ring compound of any one of claims I to 5, wherein $R^3$ is methyl or ethyl, and $Y^2$ is $C_2$-$C_3$ alkylene, or a pharmaceutically acceptable salt thereof.

**7.** The 5-membered ring compound of any one of claims 1 to 6, wherein the wavy line represents (Z)-coordination, or a pharmaceutically acceptable salt thereof.

**8.** A therapeutic agent for inflammations, comprising the 5-membered ring compound of any one of claims I to 7 or a pharmaceutically acceptable salt thereof.

**9.** A therapeutic agent for autoimmune inflammations or allergic inflammations, comprising the 5-membered ring compound of any one of claims 1 to 7 or a pharmaceutically acceptable salt thereof.

**10.** A therapeutic agent for chronic obstructive pulmonary diseases, comprising the 5-membered ring compound of any one of claims 1 to 7 or a pharmaceutically acceptable salt thereof.

**11.** A therapeutic agent for bronchial asthma, comprising the 5-membered ring compound of any one of claims I to 7 or a pharmaceutically acceptable salt thereof.

**12.** A therapeutic agent for rhinitis, comprising the 5-membered ring compound of any one of claims 1 to 7 or a pharmaceutically acceptable salt thereof.

**13.** A method for treating inflammations, comprising administering the 5-membered ring compound of any one of claims 1 to 7 or a pharmaceutically acceptable salt thereof to a patient in need thereof.

**14.** Use of the 5-membered ring compound of any one of claims 1 to 7 or a pharmaceutically acceptable salt thereof in the manufacture of a therapeutic agent for inflammations.

**INTERNATIONAL SEARCH REPORT**

International application No.
PCT/JP2009/058054

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D277/20, A61K31/426, A61K31/5377, A61P1/04, A61P3/10, A61P11/00, A61P11/02, A61P11/06, A61P17/00, A61P17/04, A61P19/02, A61P25/00, A61P27/14, A61P29/00, A61P35/04, A61P37/02, A61P37/06, A61P37/08, A61P43/00,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAPlus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2002/02542 A1 (Sumitomo Pharmaceuticals Co., Ltd.), 10 January, 2002 (10.01.02), Full text; particularly, Claims; page 17, lines 1 to 23; page 30, line 12 to page 31, line 1 & JP 2006-169257 A & US 2004/0072827 A1 & US 2005/0222226 A1 & EP 1300401 A1 & EP 1669070 A2 & AU 6634501 A & CA 2413000 A & CN 1620442 A & CN 1944417 A & KR 20070086998 A | 1,5-9,11,12, 14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 May, 2009 (19.05.09) | 02 June, 2009 (02.06.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2009/058054

C (Continuation). DOCUMENTS CONSIDERED TO BE RELEVANT

| Category* | Citation of document, with indication, where appropriate, of the relevant passages | Relevant to claim No. |
|---|---|---|
| X | JP 2003-192591 A (Sumitomo Pharmaceuticals Co., Ltd.), 09 July, 2003 (09.07.03), Full text; particularly, Claims; Par. Nos. [0024], [0046] (Family: none) | 1,5-9,11,12, 14 |
| X | WO 2006/118268 A1 (Dainippon Sumitomo Pharmaceuticals Co., Ltd.), 09 November, 2006 (09.11.06), Full text; particularly, Claims; Par. No. [0025] & EP 1875915 A1 & WO 2006/126581 A1 & CA 2603060 A & KR 20080004528 A & CN 101166534 A | 1,5-7,10 |
| A | JP 63-60978 A (Yoshitomi Pharmaceutical Industries, Ltd.), 17 March, 1988 (17.03.88), Full text; particularly, Claims; page 4, upper right column (Family: none) | 1-12,14 |

Form PCT/ISA/210 (continuation of second sheet) (April 2007)

**INTERNATIONAL SEARCH REPORT**

International application No.
PCT/JP2009/058054

Continuation of A. CLASSIFICATION OF SUBJECT MATTER
(International Patent Classification (IPC))

*C07D277/20*(2006.01)i, *A61K31/426*(2006.01)i, *A61K31/5377*(2006.01)i, *A61P1/04*(2006.01)i, *A61P3/10*(2006.01)i, *A61P11/00*(2006.01)i, *A61P11/02*(2006.01)i, *A61P11/06*(2006.01)i, *A61P17/00*(2006.01)i, *A61P17/04*(2006.01)i, *A61P19/02*(2006.01)i, *A61P25/00*(2006.01)i, *A61P27/14*(2006.01)i, *A61P29/00*(2006.01)i, *A61P35/04*(2006.01)i, *A61P37/02*(2006.01)i, *A61P37/06*(2006.01)i, *A61P37/08*(2006.01)i, *A61P43/00*(2006.01)i, *C07D277/42*(2006.01)i, *C07D417/10*(2006.01)i

(According to International Patent Classification (IPC) or to both national classification and IPC)

Continuation of B. FIELDS SEARCHED
Minimum documentation searched (International Patent Classification (IPC))

C07D277/42, C07D417/10

Minimum documentation searched (classification system followed by classification symbols)

**INTERNATIONAL SEARCH REPORT**

International application No.
PCT/JP2009/058054

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 13
because they relate to subject matter not required to be searched by this Authority, namely:
Claims 13 pertains to methods for treatment of the human body by therapy and thus relates to a subject matter which this International Searching Authority is not required, under the provisions of Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9826065 A
- WO 02002542 A
- WO 2003057693 A
- JP 2005206515 A

### Non-patent literature cited in the description


- *Synlett.,* 1997, 773-774
- *J. Org. Chem.,* 1997, vol. 62, 4539-4540
- *J. Med. Chem.,* 1984, vol. 27, 1570-1574
- *Synth. Commun.,* 1997, vol. 27, 751-756
- *J. Chem.,* 1998, 1153-1156
- *J. Chem. Soc.,* 1956, 1644-1649
- *Syn. Commun.,* 1984, 537-546
- *J. Med. Chem.,* 1987, 1497-1502
- *Tetrahedoron Lett.,* 1998, 4987-4990
- *Acta Chim. Scand.,* 1986, vol. B40, 700-702
- **Jan Runsink et al.** *Synthesis,* 1994, vol. 1, 66-72
- **Sugasawa, T. et al.** *J. Biol. Chem.,* 1997, vol. 272, 21244-21252
- **Penny A. Hutson et al.** *Am Rev Respir Dis,* 1988, vol. 137, 548-557